(19) Europäisches Patentamt / European Patent Office / Office européen des brevets

(11) **EP 2 535 402 B1**

(12) **EUROPÄISCHE PATENTSCHRIFT**

(45) Veröffentlichungstag und Bekanntmachung des Hinweises auf die Patenterteilung:
**16.04.2014 Patentblatt 2014/16**

(21) Anmeldenummer: **12172435.5**

(22) Anmeldetag: **18.06.2012**

(51) Int Cl.:
***C12M 1/107*** *(2006.01)*

(54) **Biogasanlage und Verfahren zum Betreiben einer Biogasanlage**

Biogas assembly and method for operating same

Installation de biogaz et procédé de fonctionnement d'une installation de biogaz

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.06.2011 DE 102011051135**

(43) Veröffentlichungstag der Anmeldung:
**19.12.2012 Patentblatt 2012/51**

(73) Patentinhaber: **Agraferm Technologies AG
85276 Pfaffenhofen/Ilm (DE)**

(72) Erfinder: **Kube, Jürgen, Dr.
85276 Pfaffenhofen (DE)**

(74) Vertreter: **Ganahl, Bernhard et al
PATRONUS IP
Patent- und Rechtsanwälte
Truderinger Straße 246
81825 München (DE)**

(56) Entgegenhaltungen:
WO-A1-2010/054827    DE-A1-102008 031 882
DE-T2- 68 922 151    DE-U1- 20 022 768
DE-U1-202004 020 394

**Beschreibung**

Die vorliegende Erfindung betrifft eine Biogasanlage und ein Verfahren zum Betreiben einer Biogasanlage.

Biogasanlagen weisen einen Fermenter auf, in dem organische Produkte in Biogas umgewandelt werden. Biogas besteht hauptsächlich aus Methan und Kohlendioxid. Die organischen Produkte befinden sich in der Regel in einer flüssigen oder zähflüssigen Suspension, aus welcher das Biogas gasförmig im Fermenter entweicht.

In einer EU-Richtlinie ist vorgeschrieben, dass Biogasanlagen Speicherkapazitäten für die verbrauchte Suspension von 180 Betriebstagen aufweisen müssen. Deshalb werden Biogasanlagen oftmals mit mehreren Speicherbehältern ausgestattet, die mit dem oder den Fermentern kommunizierend in Verbindung sind, so dass verbrauchte Suspension aus den Fermentern den Speicherbehältern zugeführt werden kann.

Diese Speicherbehälter sind oftmals zusätzlich als Gasspeicher ausgebildet mit einer elastischen oder beweglichen Behälterabdeckung. Diese Behälterabdeckung ist beispielsweise eine Ethylen-Propylen-Dien-Monomer-Membran (EPDM-Membran). Eine solche EPDM-Membran ist gummiartig und flexibel. Vergrößert sich die Gasmenge im Gasspeicher, dann dehnt sich die Membran aus. Hierdurch steigt der Druck unter der Membran geringfügig an.

Derartige EPDM-Membranen weisen auch eine Reihe von Nachteilen auf. So tolerieren sie nur einen geringen Überdruck von bis zu 1,5 mbar, was die richtige Einstellung von Überdrucksicherungen problematisch macht. Sie sind außerdem nur begrenzt wetterfest und können bei Stürmen beschädigt werden. Bei starkem Wind kommt es zu Druckstößen im Gasspeicher, da die Oberfläche der EPDM-Membran bewegt wird. Die Sonnenstrahlung erwärmt die Membran, wodurch die Gastemperatur im Speicher ansteigt. Als Folge dehnt sich der Gasspeicher aus. Nachts kühlt die Membran stark ab und der Gasspeicher zieht sich zusammen. Methan und Geruchsstoffe können durch die EPDM-Membran diffundieren. Es gibt auch andere Materialien, insbesondere Polymermaterialien, welche als Membran eingesetzt werden. Aber auch diese anderen Materialien weisen diese Nachteile in unterschiedlicher Intensität auf.

Derartige elastische Membranen können auch mit einem Netz oder Spannseilen kombiniert werden, die die maximale Ausdehnung der Membran beschränken. Hierdurch wird bei einem maximalen Füllstand eine weitere Ausdehnung der Membran verhindert, wodurch der Druck unter der Membran stark ansteigt.

Weiterhin sind Doppelmembrangasspeicher bekannt, die eine innere und eine äußere Membran aufweisen. Die innere Membran ist flexibel und kann verschiedene Füllstände annehmen. Die äußere Membran ist in der Regel starr und dient zum Schutz vor Wettereinflüssen, Schneelasten und UV-Strahlung.

Aus der WO 99/58458 geht ein Doppelmembrangasspeicher hervor, bei dem der Bereich zwischen den beiden Membranen mit Druckluft beaufschlagt ist.

In der DE 20 022 768 U1 ist ein Biofermenter mit einem wannenartig offenen Gärsubstratbehälter beschrieben, der mit einer gasdichten Abdeckfolie verschlossen ist, mit der er einen Gasspeicher bildet. Um die Abdeckfolie vor Witterung zu schützen, ist ein zeltplanenartiges Dach vorgesehen. Im Zentrum des Biofermenters ist ein stangenförmiges Stützelement angeordnet, das die Abdeckfolie stützt. Das Stützelement weist ein in der Länge veränderbares Auflager für die Abdeckfolie auf.

Aus der DE 10 2008 031 882 A1 geht eine Vorrichtung zur Bestimmung eines Speichervolumens eines Gasspeichers hervor. Der Gasspeicher besteht aus einem wannenförmigen, kreiszylindrischen nach oben offenen Behälter. Der Behälter ist an seiner Oberseite mit einer flexiblen Folie gasdicht abgedeckt. Über der Folie ist eine haubenartige, im wesentlichen starre Abdeckung angeordnet. Im Scheitelpunkt der starren Abdeckung befindet sich eine Lichtquelle und ein optischer Sensor zum Detektieren der Oberfläche der flexiblen Folie. Die Kontur der flexiblen Folie wird somit optisch vermessen und hieraus wird das Volumen des Gasspeichers bestimmt.

Oftmals wird auch der Druck des Biogases im Gasspeicher gemessen, der als Kopfdruck bezeichnet wird, und aus dem Kopfdruck auf den Füllungsgrad des Gasspeichers geschlossen. Es besteht jedoch ein nicht linearer Zusammenhang zwischen Füllstand und Kopfdruck, weshalb die Kopfdruckmessung zur Bestimmung des Füllstandes ungenau ist.

In der Praxis hat sich bei vielen Biogasanlagen eine Höhenmessung eines Punktes auf einer Membran des Gasspeichers etabliert. Ein solches Messverfahren ist sehr einfach und daher zuverlässig. Jedoch ist die Form der Membran nicht immer gleichmäßig, weshalb oftmals die Höhemessung eines einzelnen Punktes keinen korrekten Wert ergibt. Dies kann durch mehrere Höhenmessungen an unterschiedlichen Punkten der Membran korrigiert werden.

Es besteht ein Bedarf nach Biogasanlagen mit großer Speicherkapazität für Biogas. Eine große Speicherkapazität erlaubt das Zwischenspeichern von Biogas, wenn beispielsweise ein Gasverbraucher vorübergehend ausfällt, ohne dass das Biogas in die Umwelt abgegeben oder abgefackelt werden muss. Es gibt daher Biogasanlagen mit mehreren Speicherbehältern, die als Gasspeicher ausgebildet sind. In einem Prospekt der Firma MT-Energie GmbH (erhältlich bei http://www.mt-energie.com/mt-komponenten/mt-gasmanager) ist eine Biogasanlage mit zwei Fermenter, zwei Nachgärern und zwei Gärproduktlagern beschrieben, die jeweils als wannenförmige nach oben offene Speicherbehälter ausgebildet sind, wobei sie mit einer Doppelmembran gasdicht abgedeckt sind. Jeder dieser Behälter weist ein Gebläse auf, mit dem der Druck zwischen den beiden Membranen, der Tragluftdruck, einstellbar ist. Die Gebläse sind mit einer zentralen Steuereinrichtung verbunden. Weiterhin wird in jedem dieser Behälter der Füllstand mittels einer Kopfdruckmessung gemessen. Mit dieser zentralen Steuereinrichtung soll das Speichervolumen bei Mehrbehälteranlagen im Vergleich zu herkömmlichen Biogasanlagen besser genutzt werden. Der Gasfluss zwischen den einzelnen Behältern wird aktiv gesteuert. In den einzelnen Behältern können sich unterschiedliche Füllstände einstellen.

Aus der WO 2010/054827 A1 ist ein Gasspeichersystem für eine Biogasanlage bekannt, das zumindest zwei kaskadenartig in Reihe geschaltete Gasspeicher aufweist. Die Gasspeicher besitzen einen Zwischenraum zwischen einer Gasspeichermembran und einer Schutzabdeckung. In diesem Zwischenraum sind Druckmessgeräte vorgesehen, um den Zwischenraumdruck zu messen. Der Zwischenraumdruck kann durch eine Gaszufuhr, die in der Regel eine Luftzufuhr ist, gesteuert werden. Es ist eine zentrale Steuereinrichtung vorgesehen, die in Abhängigkeit der gemessenen Zwischenraumdrücke die Gaszufuhr zu den Zwischenräumen regelt.

Bei einer alternativen Ausführungsform der WO 2010/054827 A1 sind in den Verbindungsleitungen der Gasspeicher, die als Gasüberströmleitungen bezeichnet werden, Gasverdichter angeordnet. Die Gasverdichter können in Abhängigkeit des gemessenen Gasdrucks in Gasspeicherräumen geregelt werden. Alternativ oder zusätzlich kann eine Regelung der Gasverdichter auch jeweils auf Grundlage des gemessenen Füllstandes zumindest derjenigen Gasspeicher erfolgen, die durch die mit den jeweiligen Gasverdichtern versehene Gasüberströmleitung verbunden sind.

Diese und ähnliche Anlagen sind vielfach im Einsatz. Es hat sich in der Praxis gezeigt, dass bei der Kopplung von zwei oder mehreren Gasspeichern, wobei zumindest einer der Gasspeicher ein Doppelmembrangasspeicher mit Gebläse ist, es sehr schwierig ist, das Speichervolumen dieses Gasspeichers vollständig zu nutzen. In einem solchen Doppelmembrangasspeicher mit Gebläse wird der Tragluftdruck zwischen den Membranen durch das Gebläse eingestellt. Der Druck des Biogases im Gasspeicher entspricht im wesentlichen dem Tragluftdruck, wenn das Gewicht der inneren Membran vernachlässigt wird. Somit wird der Gasdruck des Gasspeichers durch das Gebläse vorgegeben. Ist dieser Doppelmembrangasspeicher mit Gebläse mit einem weiteren Gasspeicher verbunden, in dem ein deutlich höherer Druck vorliegt, dann fließt Biogas von dem weiteren Gasspeicher in den Doppelmembrangasspeicher, bis dieser vollständig gefüllt ist und die innere Membran gespannt ist, wodurch im Doppelmembrangasspeicher der Druck ansteigt, bis sich ein Druckgleichgewicht in beiden Gasspeichern einstellt. Ist der Doppelmembrangasspeicher mit einem Gasspeicher verbunden, in dem ein geringerer Gasdruck vorliegt, dann strömt das Biogas aus dem Doppelmembrangasspeicher in den anderen Gasspeicher bis die innere Membran auf Auflageelementen aufliegt und der Gasdruck im Doppelmembrangasspeicher abfällt, bis sich ein entsprechendes Druckgleichgewicht mit dem anderen Gasspeicher einstellt. Der Doppelmembrangasspeicher ist somit meistens entweder zu 100% gefüllt oder vollständig geleert. Hierdurch kann die Kapazität des Doppelmembrangasspeichers oftmals nicht genutzt werden, da er im Betrieb entweder immer vollständig gefüllt oder vollständig geleert ist. Dem kann entgegengewirkt werden, indem die Strömung des Biogases zwischen den beiden Gasspeichern aktiv gesteuert wird. Hierzu sind jedoch Pumpen und/oder Drosselelemente in allen Leitungsabschnitten zwischen den Fermentern und Gasspeichern notwendig, um den Druckausgleich zwischen den einzelnen Gasspeichern zu verhindern und den Füllungsgrad der einzelnen Behälter individuell einstellen zu können. Eine derartige aktive Steuerung des Gasflusses ist jedoch aufwendig, kompliziert und teuer.

Biogasanlagen mit einem Gasspeicher und einem Verbraucher werden oft so betrieben, dass der Verbraucher in Abhängigkeit vom Füllstand mit einem unterschiedlich starken Biogasstrom versorgt wird. Ist der Biogasstrom hoch, dann wird am Verbraucher, z.B. mit einem Blockheizkraft viel Biogas in Wärme und Strom umgesetzt. Ist der Füllstand gering, dann wird dem Blockheizkraftwerk weniger Biogas zugeleitet, wodurch die Produktion von Wärme und Strom reduziert wird. In der Praxis ist dies jedoch problematisch, da der gemessene Füllstand von der Temperatur abhängt und es sein kann, dass bei kalten Temperaturen in der Nacht der Füllstand alleine aufgrund der Temperaturänderung derart absinkt, dass die Zufuhr des Biogases an den Verbraucher vermindert wird. Es kann daher sein, dass obwohl genügend Biogas vorhanden ist, die Leistung des Verbrauchers zurückgefahren wird.

Der Erfindung liegt die Aufgabe zugrunde, eine Biogasanlage und ein Verfahren zum Betreiben einer Biogasanlage mit mehreren Gasspeichern zu schaffen, wobei zumindest einer der Gasspeicher als Doppelmembrangasspeicher mit Gebläse ausgebildet ist, wobei die theoretisch zur Verfügung stehende Speicherkapazität auf einfache Art und Weise zum Großteil genutzt werden kann.

Die Aufgabe wird durch eine Biogasanlage mit den Merkmalen des Anspruchs 1 und durch ein Verfahren mit den Merkmalen des Anspruchs 9 gelöst. Vorteilhafte Ausgestaltungen der Erfindung sind in den jeweiligen Unteransprüchen angegeben.

Eine weitere Aufgabe der vorliegenden Erfindung liegt in der Schaffung einer Biogasanlage und eines Verfahrens zum Betreiben der Biogasanlage so, dass der zum Verbraucher geleitete Biogasstrom entsprechend der tatsächlich vorhandenen Biogasmenge eingestellt wird.

Diese Aufgabe wird durch eine Biogasanlage mit den Merkmaien des Anspruchs 1 und durch ein Verfahren mit den Merkmalen des Anspruchs 9 gelöst. Vorteilhafte Ausgestaltungen der Erfindung sind in den jeweiligen Unteransprüchen angegeben.

Nach einem ersten Aspekt der vorliegenden Erfindung wird eine Biogasanlage mit zumindest einem Fermenter und zwei Speicherbehältern vorgesehen, wobei der Fermenter und die beiden Speicherbehälter kommunizierend miteinander verbunden sind. Die Speicherbehälter sind als Gasspeicher ausgebildet und weisen jeweils einen Füllstandssensor auf, wobei einer der Gasspeicher als Hauptgasspeicher definiert ist und der andere Gasspeicher als Nebengasspeicher definiert ist. Zumindest der Nebengasspeicher ist als Doppelmembrangasspeicher ausgebildet, welcher eine innere Membran und eine äußere Membran aufweist, wobei ein Gebläse zum Einstellen eines Tragluftdruckes zwischen den beiden Membranen vorgesehen ist.

Die erfindungsgemäße Biogasanlage zeichnet sich durch eine Steuereinrichtung aus, die mit dem Füllstandssensoren der Gasspeicher und dem Gebläse des Doppelmembrangasspeichers verbunden und derart ausgebildet ist, dass der Tragluftdruck derart eingestellt wird, dass der Füllstand im Nebengasspeicher dem Füllstand im Hauptgasspeicher entspricht.

Da der Tragluftdruck im Nebengasspeicher derart eingestellt wird, dass der Füllstand im Nebengasspeicher dem Füllstand im Hauptgasspeicher entspricht, stellt sich im Nebengasspeicher ein Druck ein, der dem Druck im Hauptgasspeicher entspricht abzüglich eines Druckabfalls in den Leitungen zwischen dem Hauptgasspeicher und dem Nebengasspeicher. Ist der Druck im Hauptgasspeicher konstant, so ist auch der Druck im Nebengasspeicher konstant, sofern der Gasstrom auch im wesentlichen konstant ist. Mit anderen Worten kann man sagen, dass sich der Druck im Nebengasspeicher derart einstellt, dass der Nebengasspeicher zusammen mit dem Hauptgasspeicher "atmet". Wenn der Hauptgasspeicher auf seine minimale Gasmenge reduziert wird, dann wird dementsprechend auch der Nebengasspeicher auf seine minimale Gasmenge reduziert. Wird der Hauptgasspeicher vollständig gefüllt, dann wird auch gleichzeitig der Nebengasspeicher vollständig gefüllt.

Da der Druck im Nebengasspeicher dem Druck im Hauptgasspeicher folgt und dieser in der Regel konstant ist, kann die Regelung des Druckes im Nebengasspeicher mit einem einfachen Regelmechanismus, wie z.B. einer Zwei-Punkt-Regelung oder einem Proportionalregler zuverlässig und präzise ausgeführt werden. Selbstverständlich ist es auch möglich einen PI- oder PID-Regelmechanismus zu verwenden, wobei P einen Proportionalregler, I einen Intergralregler und D einen Differentialregler bedeutet.

Bei der Erfindungsgemäßen Biogasanlage steht die Speicherkapazität des Hauptgasspeichers und des Nebengasspeichers somit vollständig zur Verfügung. Es ist nicht notwendig, dass in den Leitungen zwischen den Gasspeichern oder zwischen dem Fermenter und dem Gasspeicher Pumpen und/oder Drosseln vorgesehen werden, die aktiv den Gasstrom beeinflussen. Die Verbindungsleitungen zwischen den Gasspeichern können somit frei von die Strömungsgeschwindigkeit beeinflussenden Elementen, wie z.B. Pumpen oder Drosseln, sein. Wenn die Biogasanlage mehrere Fermenter und mehrere Gasspeicher aufweist, die mit einem Netz von Leitungen miteinander verbunden sind, in dem die Flüsse zwischen den einzelnen Behältern unterschiedlich durch Schließen und Öffnen von Ventilen geschaltet werden können, dann stellt sich nach dem Schalten eines Ventils automatisch der Druck im Nebengasspeicher auf die geänderten Strömungsverhältnisse ein.

Vorzugsweise umfasst die Biogasanlage mehrere Nebengasspeicher die untereinander und mit einem oder mehreren Fermenter kommunizierend verbunden sein können. In allen Nebengasspeichern wird der Tragluftdruck derart eingestellt, dass der Füllstand in den jeweiligen Nebengasspeichern dem Füllstand im Hauptgasspeicher entspricht.

In der Biogasanlage kann ein Verbraucher, wie z.B. ein Blockheizkraftwerk, vorgesehen sein. Der Verbraucher ist zumindest mit einem der Gasspeicher kommunizierend verbunden, damit dem Verbraucher Biogas zugeführt werden kann. Der Druck im Hauptgasspeicher wird vorzugsweise derart eingestellt, dass das Biogas am Verbraucher mit einem geringfügig größeren Druck als dem Atmosphärendruck anliegt. Am Verbraucher beträgt der Druck des Biogases vorzugsweise etwa 0,1 mbar bis 2 mbar über dem Atmosphärendruck. Der Druck im Hauptgasspeicher kann derart auf einen konstanten Wert eingestellt werden, dass sichergestellt ist, dass dem Verbraucher zuverlässig Biogas zugeführt wird.

Der Füllstandssensor des Doppelmembrangasspeichers ist vorzugsweise zum Detektieren der Höhe eines bestimmten Punktes der inneren Membran ausgebildet. Nach einer bevorzugten Ausführungsform ist die innere Membran mit einem Gewicht beaufschlagt, wodurch die Messung der Höhe eines bestimmten Punktes der inneren Membran besser mit dem Füllstand des Gasspeicher korreliert.

Die erfindungsgemäße Biogasanlage bzw. das erfindungsgemäße Verfahren zum Betreiben einer Biogasanlage mit mehreren Gasspeichern ist sehr einfach ausgebildet und erlaubt einen stabilen Betrieb. Dies zeigt insbesondere ein Vergleich mit den aus der WO 2010/054827 A1 bekannten Biogasanlagen. Bei der ersten oben erläuterten Ausführungsform nach der WO 2010/054827 A1 wird der Zwischenraumdruck (= Tragluftdruck) der einzelnen Gasspeicher gemessen und die Zwischenraumdrücke werden durch Nachführen von Luft mittels einer zentralen Steuereinrichtung geregelt. Bei den heutzutage üblichen, in der Regel mit einer etwa halbkugelförmigen Schutzabdeckung versehenen Biogasspeichern, ist der Druck im Biogasspeicher bzw. im Zwischenraum bei einem Füllstand von 20% bis 80% des maximalen Füllstandes im wesentlichen unabhängig vom Füllstand. Daher hat eine solche Druckregelung nur sehr bedingt Einfluss auf den tatsächlichen Füllstand im jeweiligen Biogasspeicher. Eine solche Druckregelung ist sehr einfach und zuverlässig, solange die zeitliche Änderung des Gasvolumens in einem Gasspeicher gering ist. Bei einem Verbraucher mit stark schwankendem Biogasbedarf kann dies jedoch zur Folge haben, dass der unmittelbar an den Verbraucher angeschlossene Biogasspeicher fast vollständig entleert wird, wohingegen die weiteren Biogasspeicher noch fast vollständig gefüllt bleiben. Dies kann zu Problemen beim Versorgen des Verbrauchers mit Biogas führen. Bei der erfindungsgemäßen Biogasanlage wird hingegen aus allen Biogasspeichern gleichmäßig Biogas abgezogen, auch wenn der Verbrauch an Biogas kurzfristig stark ansteigt.

Aus der WO 2010/054827 A1 ist eine alternative Ausführungsform bekannt, die oben bereits kurz erläutert ist, bei welcher Gasverdichter in den Gasüberströmleitungen zwischen den einzelnen Gasspeichern vorgesehen sind. Mit solchen Gasverdichtern kann ein schneller und kontrollierter Gasaustausch zwischen den einzelnen Gasspeichern erfolgen, so dass mit einer solchen Biogasanlage auch schnell auf einen sich verändernden Bedarf reagiert werden kann. Wenn jedoch ein solcher Verdichter, der in der Regel als Gebläse ausgebildet ist, ausfällt, dann bildet er einen erheblichen Strömungswiderstand in der Leitung. Dies beeinträchtigt wiederum den Gasaustausch zwischen den daran angeschlossenen Gasspeichern erheblich. Zudem sind bei Verwendung derartiger Gasverdichter die Leitungen sehr klein dimensioniert, wodurch bei einem Ausfall des Gasverdichters der Strömungswiderstand umso größer wird. Ein Ausfall eines Gasverdichters kann zu einem unkontrolliert hohen Druckanstieg in einem Speicher und zu einem Druckabfall mit Unterdruck in dem anderen Speicher führen. Bei Unterdruck kann Luft von außen angesaugt werden, die sich mit dem Biogas vermischt und ein hochexplosives Gemisch darstellt. Daher sind bei Verwendung von Verdichtern in der Regel zwei Verdichter in einer Leitung anzuordnen, um das System redundant zu halten. Bei mehreren Biogasspeichern, die alle miteinander verbunden sind, kann es daher notwendig sein, eine Vielzahl derartiger Verdichter vorzusehen. Dies macht das ganze System aufwendig, komplex und teuer.

Mit der Erfindung wird dies vermieden, da der Tragluftdruck der einzelnen Gasspeicher derart eingestellt wird, dass der Füllstand im Nebengasspeicher dem Füllstand im Hauptgasspeicher entspricht, wobei mit einfachen zuverlässigen Füllstandssensoren der Füllstand gemessen werden kann.

Nach einem zweiten Aspekt der vorliegenden Erfindung ist eine Biogasanlage vorgesehen, die zumindest einen Gasspeicher und einen Verbraucher aufweist, der mit dem Gasspeicher derart kommunizierend verbunden ist, dass dem Verbraucher Biogas zugeführt werden kann. Die Biogasanlage weist eine Steuereinrichtung zum Steuern der Strömungsmenge des dem Verbraucher zuzuführenden Biogases auf. Die Strömungsmenge wird in Abhängigkeit einer temperaturkorrigierten Füllmenge des Gasspeichers gesteuert. Hierdurch wird sichergestellt, dass aufgrund von Temperaturschwankungen, die zu einer Änderung des tatsächlichen Füllstandes führen, keine Fehlsteuerung verursacht wird. Ist der Verbraucher ein Blockheizkraft mit einem Gasmotor, dann wird die Strömungsmenge, die vom Gasspeicher zum Verbraucher übertragen wird, durch den Verbrauch am Gasmotor gesteuert.

Vorzugsweise wird ein Temperatursensor zum Messen der Gastemperatur verwendet, der im Gasspeicher oder in einer den Gasspeicher mit dem Verbraucher verbindenden Leitung angeordnet ist.

Der Messzeitraum kann einen oder mehrere Tage (z.B. 3-15 Tage, insbesondere 5-10 Tage) betragen.

Die beiden oben beschriebenen Aspekte der vorliegenden Erfindung können auch kombiniert werden, wobei eine Biogasanlage mit mehreren Gasspeichern vorgesehen ist, und eine temperaturkorrigierte Füllmenge aller Gasspeicher ermittelt wird, anhand der die Strömungsmenge des Biogases zum Verbraucher gesteuert wird.

Gasspeicher sind Speicherbehälter, die zum Aufnehmen unterschiedlicher Gasmengen ausgebildet sind, insbesondere sind Gasspeicher nach oben offene wannenförmige Behälter mit einer beweglichen, gasdichten Abdeckung.

Der Erfindung wird nachfolgend beispielhaft anhand der Zeichnungen erläutert. Die Zeichnungen zeigen in:

| | |
|---|---|
| Figur 1 | schematisch in einem Blockschaltbild eine Biogasanlage mit zwei Fermentern und drei Gasspeichern, |
| Figur 2 | die Druck- und Strömungsverhältnisse des Biogases in der Biogasanlage nach Figur 1, wobei alle Verbindungsleitungen geöffnet sind, |
| Figur 3 | die Druck- und Strömungsverhältnisse des Biogases in der Biogasanlage nach Figur 1, wobei nur bestimmte Verbindungsleitungen geöffnet sind, |
| Figur 4a bis 4h | Doppelmembranspeicher schematisch vereinfacht in verschiedenen Ausführungsformen und mit verschiedenen Füllständen, |
| Figur 5 | schematisch unterschiedliche Volumensegmente eines Biogasspeichers mit Doppelmembran, |
| Figur 6 | ein Diagramm eines temperaturkorrigierten Füllstandssignals bei unterschiedlichen Temperaturen, |
| Figur 7 | in einem weiteren Diagramm einen beispielhaften Zusammenhang zwischen einem temperaturkorrigierten Füllstandsignal und der Auslastung des Gasverbrauchers, |
| Figur 8 | ein Diagramm einer Zweipunktregelung, |
| Figur 9 | ein Diagramm einer Zweipunktregelung mit einem FU-Gebläse, und |
| Figur 10 | ein Diagramm der Tragluftdrücke mit einer Zweipunktregelung mit kurzen Ausschaltzeiten. |

Eine erfindungsgemäße Biogasanlage weist zumindest einen Fermenter 1 und zwei Speicherbehälter 2 auf. Die Speicherbehälter 2 sind als Gasspeicher 2 ausgebildet.

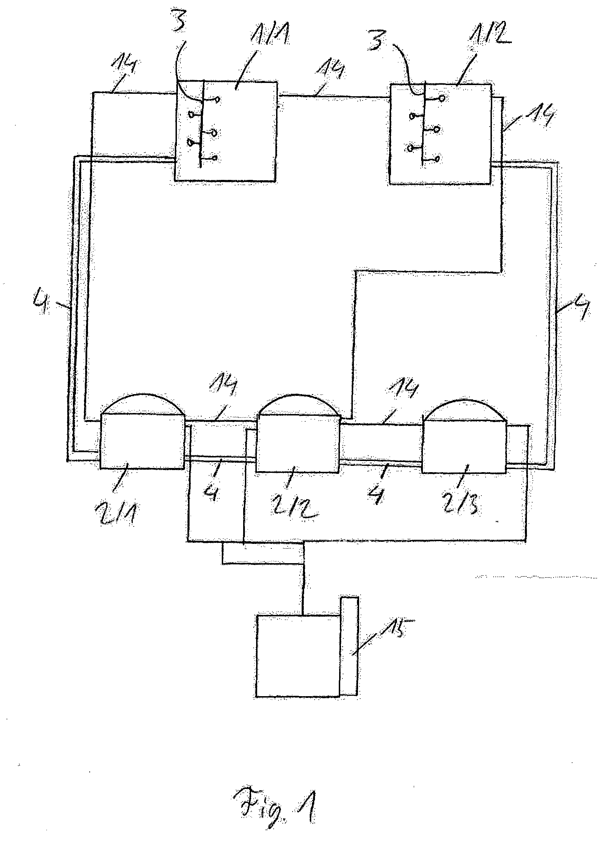

Figur 1 zeigt ein Ausführungsbeispiel einer erfindungsgemäßen Biogasanlage mit zwei Fermentern 1/1, 1/2 und drei als Gasspeicher ausgebildete Speicherbehälter 2/1, 2/2, 2/3.

Die Fermenter 1 sind wannenförmige nach oben hin geschlossene Behälter zur Aufnahme einer Suspension aus Biomasse. In den Fermentern 1 wird die Biomasse in Biogas umgesetzt. Die Fermenter 1 sind jeweils mit einem Rührwerk 3 zum Umwälzen der Biomasse enthaltenen Suspension im Fermenter 1 versehen.

Die Speicherbehälter 2 dienen zum Aufnehmen von verbrauchter Suspension der Fermenter 1. Die Speicherbehälter 2 sind deshalb mit den beiden Fermentern 1 über ein Suspensionsrohrleitungsnetzwerk 4 verbunden über welches die verbrauchte Suspension von den Fermentern 1 zu den Speicherbehältern 2 geleitet werden kann.

Von den Fermentern 1/1 und 1/2 führt jeweils eine Suspensionsrohrleitung 4 zu den Speicherbehältern 2/1 und 2/3. Die beiden Speicherbehälter 2/1 und 2/3 dienen bezüglich der Fermenter 1/1 und 1/2 als Nachgärer, in welchen die Biomasse nach dem Hauptgärprozess in den Fermentern 1/1 und 1/2 noch weiter vergären kann und Biogas erzeugt. Die Speicherbehälter 2/1 und 2/3 sind jeweils mit einer Suspensionsrohrleitung 4 mit dem Speicherbehälter 2/2 verbunden, über die die Suspension aus den beiden Speicherbehältern 2/1 bzw. 2/3 an den Speicherbehälter 2/2 weitergeleitet werden kann.

Aus den Speicherbehältern 2/1 bis 2/3 kann die verbrauchte Suspension entnommen werden und beispielsweise als Gülle zum Düngen von Feldern verwendet werden.

Die Speicherbehälter 2/1 bis 2/3 sind nicht nur zum Speichern von verbrauchter Suspension der Fermenter vorgesehen, sondern auch als Gasspeicher ausgebildet. Diese Speicherbehälter sind nach oben offene Wannen, die in der Regel in der Draufsicht einen kreisförmigen Querschnitt besitzen. Sie sind nach oben hin mit einer beweglichen Membran abgedeckt. Im vorliegenden Ausführungsbeispiel sind alle drei Speicherbehälter 2/1 bis 2/3 als Doppelmembrangasspeicher mit Gebläse zum Einstellen eines Tragluftdruckes zwischen den beiden Membranen ausgebildet.

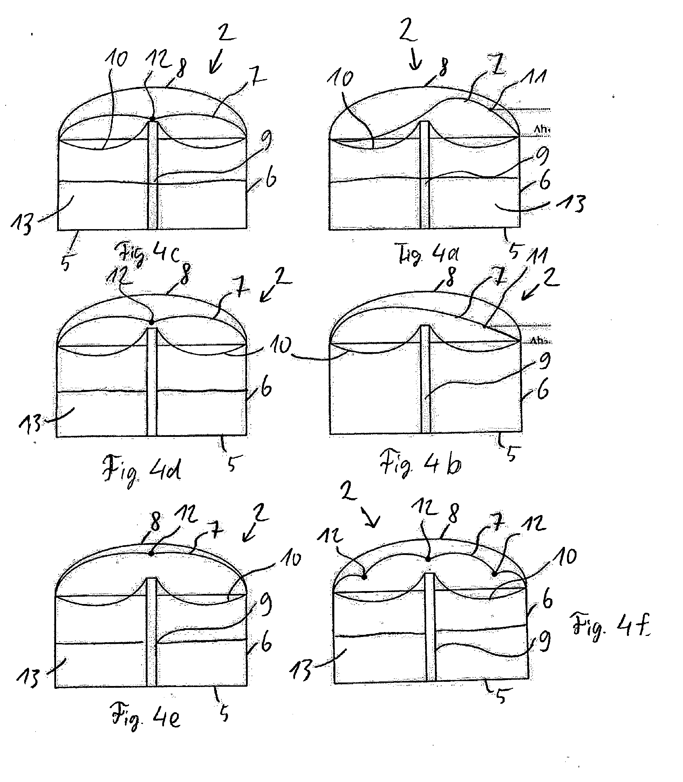

In den Figuren Figur 4a bis 4h sind schematisch Doppelmembrangasspeicher in unterschiedlichen Ausführungsformen und mit unterschiedlichen Füllständen gezeigt. Diese Doppelmembrangasspeicher weisen jeweils eine in der Draufsicht kreisförmige Bodenwandung 5, eine zylinderförmige Mantelwandung 6, eine innere Membran 7, die bündig am oberen Rand der zylinderförmigen Mantelwandung 6 befestigt ist und eine äußere, nicht dehnbare Membran 8, die oberhalb der inneren Membran 7 angeordnet und auch mit dem oberen Rand der zylinderförmigen Mantelwandung 6 verbunden ist. Die innere Membran 7 kann aus einem elastischen dehnbaren Material ausgebildet sein. Diese Doppelmembrangasspeicher weisen ein Gebläse (nicht dargestellt) auf, um Luft mit einem vorbestimmten Tragluftdruck in den Bereich zwischen der inneren Membran 7 und der äußeren Membran 8 zu pumpen. Hierdurch wird sichergestellt, dass die äußere Membran 8 konvex nach oben gewölbt ist, so dass sie ein stabiles, kuppelförmiges Dach für den Speicherbehälter bildet.

Eine Mittelsäule ist mittig im Gasspeicher 2 vertikal stehend angeordnet. Sie ragt mit ihrem oberen Ende etwas über den oberen Rand der Mantelwandung 6 hinaus. Vom oberen Ende der Mittelsäule 9 erstrecken sich Spanngurte 10 radial nach außen zum oberen Rand der Mantelwandung 6. Die Spanngurte 10 dienen als Auflagen für die innere Membran 7 bei einer geringen Füllung mit Biogas, um zu verhindern, dass die innere Membran 7 auf der Suspension aus Biomasse aufliegt. Die innere Membran 7 kann aber auch auf dem oberen Ende der Mittelsäule 9 bei geringem Füllstand zur Auflage kommen. Diese Spanngurte können straff gespannt sein, aber auch etwas lose durchhängen, wie es in den Fig. 4a bis 4h gezeigt ist.

Figur 4a und Figur 4b zeigen jeweils einen Speicherbehälter identischer Ausführung mit gleichem Füllstand. Die innere Membran 7 kann aufliegend auf einer Blase aus Biogas in Schwingung geraten und so unterschiedliche Formen annehmen. Figur 4a und 4b zeigen zwei entsprechend unterschiedliche Formen der inneren Membran 7. Auf der inneren Membran 7 ist schematisch ein Messpunkt 11 für eine Messung der Höhe der Membran eingezeichnet. Die Höhe der Membran 7 wird als inneres Maß für den Füllstand des Gasspeichers 2 verwendet. Obwohl der Füllstand in Figur 4a und 4b identisch ist, befindet sich der Messpunkt 11 auf unterschiedlichen Höhen, so dass ein unterschiedlicher Füllstand gemessen wird. Um derartige Abweichungen zu vermeiden, ist es zweckmäßig, die innere Membran 7 mit einem oder mehreren Gewichten 12 zu versehen, die Turbulenzen im Biogas dämpfen und sicherstellen, dass die innere Membran 7 in Abhängigkeit vom Füllstand eine eindeutige Form und Stellung einnimmt (Fig. 4c bis 4f). Hierdurch ergibt die Messung der Höhe eines bestimmten Punktes der inneren Membran 7 eine zuverlässige Auskunft über den Füllstand des Gasspeichers 2.

Die Höhenmessung eines Punktes auf der inneren Membran des Gasspeichers kann beispielsweise mittels eines Seillängengebers erfolgen. Hierzu wird ein Seil an dem Messpunkt 11 der inneren Membran 7 des Gasspeichers 2 befestigt und zum Rand des Gasspeichers geleitet. Füllt sich der Gasspeicher, dann ändert der Messpunkt 11 seine Position. Die Auslenkung des Seils dient als Füllstandssignal.

Eine andere Möglichkeit der Messung der Höhe der inneren Membran ist das Vorsehen eines mit einer Flüssigkeit gefüllten Schlauches an der inneren Membran. Am unteren Ende des Schlauches wird der hydrostatische Druck gemessen. In Abhängigkeit von der Höhe des Schlauches ändert sich der hydrostatische Druck. Die Druckmessung erfolgt mit einem elektronischen Drucksensor, bei dem die Höhe der inneren Membran 7 bzw. der Füllstand des Gasspeichers 2 als elektrisches Signal vorliegt, das einer elektronische Steuereinrichtung zugeführt werden kann.

Selbstverständlich sind auch noch andere Messmethoden, insbesondere optische Messmethoden möglich, wie sie z.B. in der DE 10 2008 031 882 A1 beschrieben sind.

Die Höhenmessung eines Punktes der inneren Membran ist eine eindeutige und zuverlässige Methode zur Bestimmung des von der inneren Membran aufgespannten Volumens oberhalb des oberen Randes der Mantelwandung 6.

Der Füllstand der Suspension wird wie an sich bekannter Weise mit einem weiteren Füllstandssensor gemessen, so dass das weitere vom Biogas im Gasspeicher 2 eingenommene Volumen zwischen der Oberfläche der Suspension 13 und dem oberen Rand der Mantelwandung 6 bekannt ist.

Ein jeder Gasspeicher 2 weist einen Füllstandssensor zum Messen des Füllstandes des Gases im Gasspeicher auf. Da der Füllstand des Gases auch vom Füllstand der Suspension im Gasspeicher abhängt, umfasst der Füllstandssensor zum Messen des Gasvolumens im Gasspeicher einen Sensor zum Messen des Ortes der inneren Membran 7 als auch einen Sensor zum Messen des Füllstandes der Suspension und eine Auswerteeinrichtung, mit welcher das gesamte Volumen des Biogases im Gasspeicher 2 ermittelt wird. Sofern nichts anderes gesagt ist, bedeutet der Begriff "Füllstandssensor" eine solche Einrichtung mit zwei Sensoren zum Bestimmen des tatsächlichen Gasvolumens des Biogases im Gasspeicher.

Die Biogasanlage weist eine Steuereinrichtung (nicht dargestellt) auf, die mit einem jeden Füllstandssensor der Gasspeicher 2 und den Gebläsen zum Erzeugen des Tragluftdruckes in der Doppelmembran verbunden ist.

Die Fermenter 1 und die Gasspeicher 2 sind mit Gasleitungen 14 derart verbunden, dass das in einem Fermenter 1 erzeugte Biogas zumindest einem der Gasspeicher 2 zugeführt werden kann, wobei die Fermenter 1 und die Gasspeicher 2 auch untereinander mittels Gasleitungen 14 verbunden sind. Es besteht somit ein Gasleitungsnetz, in dem das in einem der Fermenter erzeugte Biogas auf unterschiedlichen Wegen geführt werden kann. Im Gasleitungsnetz sind Ventile (nicht dargestellt) angeordnet, mit welchen einzelne Gasleitungen 14 geöffnet und geschlossen werden können, sodass unterschiedliche Strömungswege im Gasleitungsnetzwerk einstellbar sind. Von den Gasspeichern 2 führen weitere Gasleitungen 14 zu einem Verbraucher, der im vorliegenden Ausführungsbeispiel ein Blockheizkraftwerk 15 ist. Der Verbraucher kann auch eine Einspeisestation zum Einspeisen von Bioerdgas in ein Erdgasnetzwerk oder ein Gasbrenner sein, um Wärme zu erzeugen.

Erfindungsgemäß wird einer Gasspeicher 2/1 als Hauptgasspeicher und der oder die anderen Gasspeicher 2/2, 2/3 als Nebengasspeicher definiert.

Die Steuereinrichtung ist derart ausgebildet, dass der Tragluftdruck in dem bzw. den Nebengasspeichern derart eingestellt wird, dass der Füllstand an Biogas in dem jeweiligen Nebengasspeicher 2 dem Füllstand im Hauptgasspeicher entspricht.

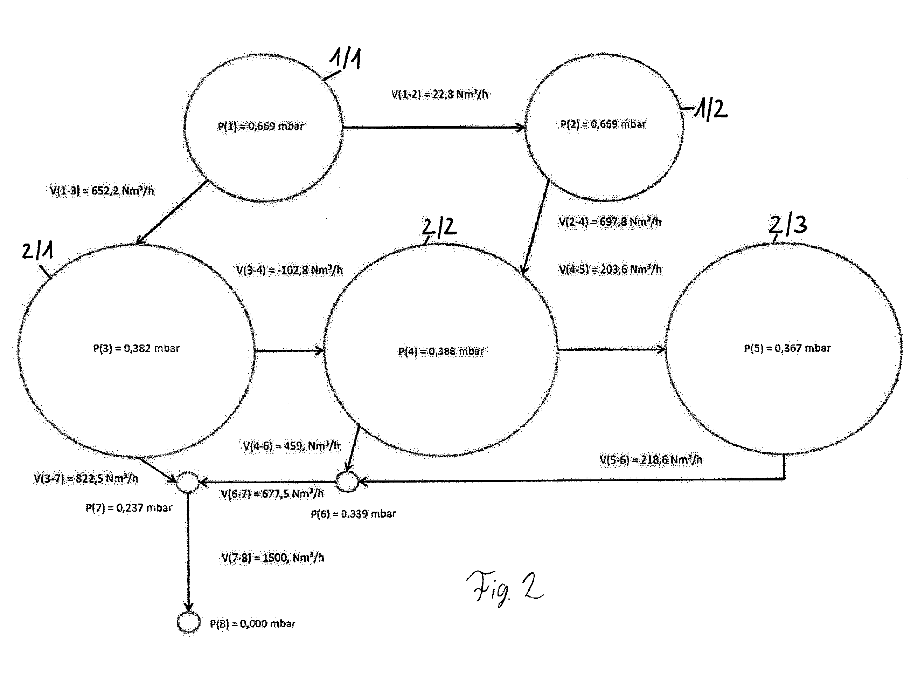

Figur 2 zeigt schematisch in einem Blockschaltbild die Biogasanlage aus Figur 1, wobei alle Gasleitungen 14 geöffnet sind. In Figur 2 sind an bestimmten Punkten Druckwerte und die Gasströmungen zwischen den Punkten angeben. Die Punkte sind mit den Zahlen 1 bis 8 nummeriert, wobei den Zahlen jeweils folgender Ort zugeordnet ist:

1    Fermenter 1/1
2    Fermenter 1/2
3    Gasspeicher 2/1 (Hauptgasspeicher)
4    Gasspeicher 2/2 (Nebengasspeicher)
5    Gasspeicher 2/3 (Nebengasspeicher)
6    Knotenpunkt der Gasleitungen von den Gasspeichern 2/2 und 2/3 mit einer Leitung zu einem weiteren Knotenpunkt
7    Ein Knotenpunkt einer Gasleitung 14 vom Gasspeicher 2/1 mit einer Gasleitung vom oben beschriebenen Knotenpunkt 6 und einer zum Blockheizkraftwerk 15 führenden Gasleitung.
8    Der Anschlusspunkt einer der Gasleitungen am Blockheizkraftwerk 15.

In Figur 2 sind Druckwerte P(n) angegeben, wobei für n die Ziffer eines der Punkte 1 bis 8 eingesetzt ist. So gibt z.B. der Druckwert P(1) den Druckwert im Fermenter 1/1 an. Die in Figur 2 dargestellten Druckwerte sind alles Druckwerte über dem Atmosphärendruck. Weiterhin sind in Figur 2 Strömungswerte V(n-i) angegeben, wobei n und i jeweils die Zahlen der acht Punkte sind, sodass die Strömungswerte jeweils die Gasströmung zwischen dem Punkt n und i angeben. Beispielsweise bedeutet der Strömungswert V(1-2) den Strömungswert der Gasströmung vom Fermenter 1/1 zum Fermenter 1/2. Bei einer Strömung durch eine Rohrleitung gibt es aufgrund der Reibung des Gases im Rohr einen Druckverlust. Dieser Druckverlust kann in Abhängigkeit der Strömungsgeschwindigkeit des Gases im Rohr, der Länge des Rohres, des Innendurchmessers des Rohres, der Dichte des Gases bei Betriebsbedingungen und der Rohrreibungszahl berechnet werden. Aufgrund dieses Druckverlustes ist in einem solchen System kommunizierender Behälter, in dem das Gas nicht aktiv durch Pumpen oder dergleichen befördert wird, der Druck des Gases vom Fermenter kommend in dem das Biogas entsteht, bis zum Verbraucher zunehmend geringer. Damit überhaupt Biogas an den Verbraucher gelangen kann, muss an der Anschlussstelle des Gasleitungsnetzwerkes 14 am Verbraucher 15 ein Druck von zumindest dem Atmosphärendruck (= P(8) =0,000 mbar) bestehen. Dies ist der geforderte Mindestdruck. Ausgehend von dieser Mindestanforderung wurden für die Biogasanlage aus Figur 1 die in Figur 2 aufgeführten Druck- und Strömungswerte für den Zustand berechnet, bei dem alle Gasleitungen 14 geöffnet sind. Es ergaben sich Druckwerte von 0,669 mbar für die beiden Fermenter 1/1 und 1/2 und Druckwerte von 0,382 mbar für den Gasspeicher 2/1, von 0,388 mbar für den Gasspeicher 2/2 und 0,367 mbar für den Gasspeicher 2/3. Da bei dieser Schaltung die Gasspeicher im wesentlichen parallel geschaltet sind, weisen sie ähnliche Druckwerte auf.

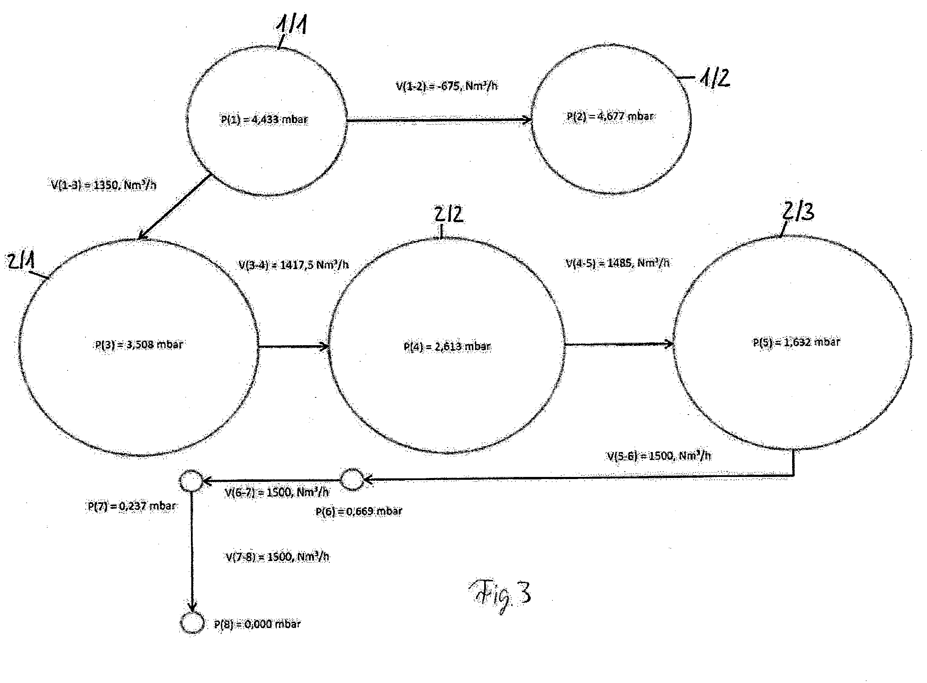

Figur 3 zeigt die gleiche Anordnung, wobei jedoch einzelne Gasleitungen abgeschaltet sind, sodass lediglich ein einziger Strömungsweg besteht, der vom Fermenter 1/2 zum Fermenter 1/1 und von dort zum Gasspeicher 2/1, dann zum Gasspeicher 2/2 und dann zum Gasspeicher 2/3 führt. Vom Gasspeicher 2/3 führt der Strömungsweg zum Verbraucher 15. Aufgrund des Druckabfalls in den einzelnen Abschnitten der Gasleitung 14 ergibt sich bei einen Atmosphärendruck am Verbraucher 15 (P (8) = 0,000 mbar) am Fermenter 1/2 ein Druck von 4,677 mbar, am Fermenter 1/1 ein Druck von 4,433 mbar, am Gasspeicher 2/1 ein Druck von 3,508 mbar, am Gasspeicher 2/2 ein Druck von 2,613 mbar und am Gasspeicher 2/3 ein Druck von 1,632 mbar.

Im Vergleich zu der Schaltung von Figur 2 ist bei der Schaltung aus Figur 3 der Druck in den Fermentern wesentlich höher.

Gasspeicher besitzen in der Regel ein Druckbegrenzungsventil. Bei Doppelmembrangasspeichern beträgt der Schwellwert des Druckbegrenzungsventils in der Regel 2 mbar, ab einem Druck von 7 mbar wird Biogas automatisch an die Atmosphäre abgeleitet. Damit im Normalbetrieb kein Biogas an die Umgebung abgegeben wird, sollte der Druck im Normalbetrieb auf maximal 5 mbar begrenzt sein. Bei der Schaltung gemäß Figur 3 wird vorzugsweise der Gasspeicher 2/1 als Hauptgasspeicher definiert, da dies der Gasspeicher mit dem höchsten Druck ist. Die beiden weiteren Gasspeicher 2/2 und 2/3 werden als Nebengasspeicher definiert. Im Hauptgasspeicher wird der Druck vorzugsweise auf etwa 4,5 mbar eingestellt, wodurch er unter dem Schwellwert des Druckbegrenzungsventils liegt. Hierdurch werden auch die weiteren Druckwerte gegenüber dem in Figur 3 gezeigten Werten um etwa 1 mbar erhöht, sodass an der Anschlussstelle zum Blockheizkraftwerk 15 das Biogas mit einem Druck von etwa 1 mbar über Atmosphärendruck dem Blockheizkraftwerk zugeführt wird. In den Fermentern 1/1 und 1/2, die in der Regel eine starre Decke aufweisen, können im Vergleich zu den Gasspeichern höhere Druckwerte vorliegen. In der Praxis können in Fermentern Druckwerte bis zu 10 mbar ohne weiteres gehalten werden.

Bei der erfindungsgemäßen Regelung des Druckes ist die Regelgröße nicht der Druck, sondern der Füllstand im Nebengasspeicher, wobei als Sollgröße der Füllstand im Hauptgasspeicher vorgegeben wird. Hierbei stellt sich jedoch im Nebengasspeicher ein Druck ein, der dem Druck im Hauptgasspeicher abzüglich des Druckabfalles zwischen den beiden Gasspeichern entspricht. Werden einzelne Gasleitungen 14 geöffnet oder geschlossen, dann ändert sich der Strömungsweg und damit auch die Druckabfälle zwischen den einzelnen Gasspeichern. Mit der erfindungsgemäßen Regelung stellt sich bei einer solchen Änderung des Strömungsweges die Drücke der einzelnen Gasspeicher wieder so ein, dass sie unter Berücksichtigung der Druckabfälle in den Gasleitungen wieder im Gleichgewicht zueinander sind. Die Stellgröße des erfindungsgemäßen Regelverfahrens ist der Tragluftdruck. Der Druck in den einzelnen Gasspeichern wird somit indirekt geregelt.

Wird der Druck im Hauptgasspeicher 2/1 auf 4,5 mbar eingestellt und von dem Schaltungszustand gemäß Figur 3 auf den Schaltungszustand gemäß Figur 2 gewechselt, dann wird auch in keinem der weiteren Gasspeicher 2/2 und 2/3 der Grenzwert von 5 mbar überschritten. Am Verbraucher, dem Blockheizkraftwerk 15, liegt dann das Biogas mit einem Druck von etwas mehr als 4 mbar an.

Bei dem erfindungsgemäßen Ausführungsbeispiel ist es nicht möglich, bei einer Vorgabe des Druckes im Hauptgasspeicher 2/1 von 4,5 mbar das Leitungsnetz 14 so zu schalten, dass in einem der Nebengasspeicher ein Druck über dem Grenzwert von 5 mbar entsteht, außer wenn der Hauptgasspeicher vollständig aus dem Strömungsweg des Biogases ausgeschlossen werden würde, was keinen Sinn macht.

Ein wesentlicher Vorteil der vorliegenden Erfindung liegt darin, dass alle Gasspeicher gleichmäßig je nach Füllungsgrad mit Biogas gefüllt sind und als Durchmischungsraum für das Biogas dienen. Die Produktion des Biogases ist nicht immer konstant, so wird beispielsweise beim Einschalten eines Rührwerkes viel Biogas auf einmal freigesetzt. Hierdurch erhöht sich im Fermenter der Druck kurzzeitig. Auf derartige Druckspitzen können von der erfindungsgemäßen Regelung leicht aufgenommen werden und führen zu einem kurzzeitigen Anheben des Druckes in allen Gasspeichern.

Es ist üblich den Verbraucher einer Biogasanlage in Abhängigkeit von der vorhandenen Gasmenge zu steuern. Ist in der Biogasanlage viel Gas gespeichert, dann wird der Verbraucher bei einer höheren Last betrieben, so dass mehr Biogas verbraucht wird als wenn wenig Biogas in der Biogasanlage gespeichert ist. Hierfür gibt es unterschiedliche Gründe. Zum einen möchte man nicht, dass die Gasspeicherkapazitäten immer in der Biogasanlage vollständig ausgeschöpft sind, denn wenn weiteres Biogas in einem solchen Zustand hergestellt werden sollte und nicht sofort verbraucht werden kann, dann müsste es in die Umgebung abgegeben werden. Andererseits möchte man oftmals den Verbrauch kontinuierlich betreiben und nicht vollständig abschalten müssen, weshalb es zweckmäßig ist, bei einem geringen Speichervolumen den Verbrauch an weiterem Biogas zu verringern. Da sich aufgrund von Temperaturschwankungen das tatsächlich gespeicherte Biogasvolumen erheblich verändern kann, führt eine automatische Regelung eines Verbrauchers oftmals dazu, dass die Leistung, mit der der Verbraucher betrieben wird, im wesentlichen von der Umgebungstemperatur und nicht von der tatsächlich vorliegenden Biogasmenge bestimmt wird.

Eine sehr einfache Möglichkeit zur Realisierung der Regelung ist eine sogenannte Zweipunktregelung mit Totband. Bei einer solchen Zweipunktregelung mit Totband gilt grundsätzlich, dass das Stellglied zwei Zustände annehmen kann (z.B. an/aus oder 100% / 50%). Ist die Regelgröße kleiner als die Führungsgröße minus Totband, schaltet der Regler die Stellgröße so, dass die Regelgröße wieder zu steigen beginnt. Ist die Regelgröße größer als die Führungsgröße plus Totband, schaltet der Regler die Stellgröße so, dass die Regelgröße wieder zu sinken beginnt. Wenn der Betrag der Regelabweichung kleiner als das Totband ist, schaltet der Regler nicht.

Die Regelabweichung ist Führungsgröße (im Ausführungsbeispiel x1) minus Regelgröße (im Ausführungsbeispiel x2).

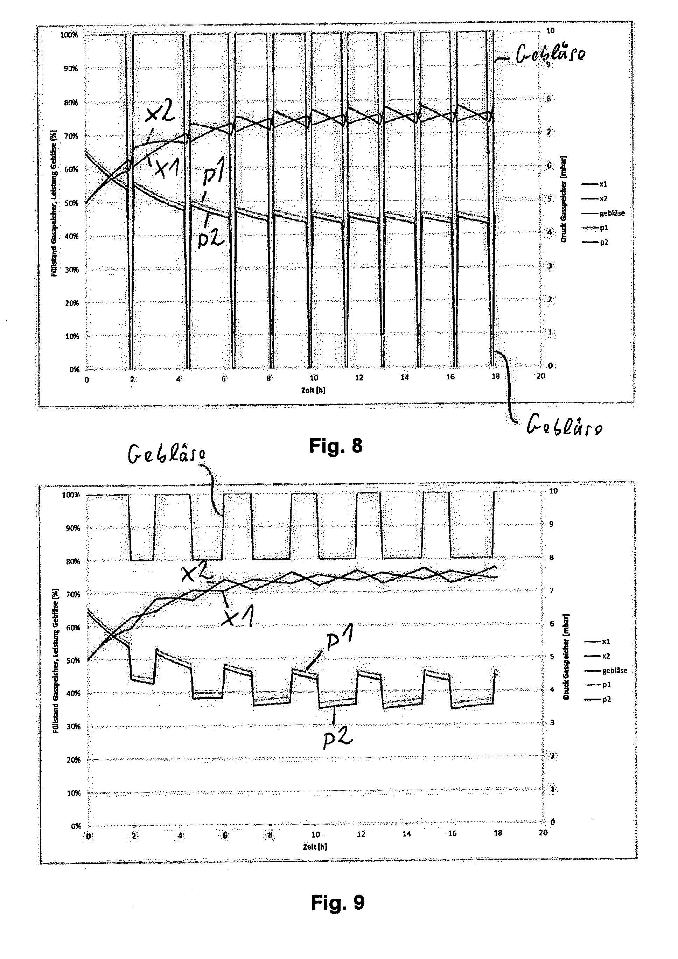

Werden die Gebläse zum Einstellen des Tragluftdruckes im Nebengasspeicher mit einer Zweipunktregelung an- und ausgeschaltet, ergibt sich der in Figur 8 gezeigte Verlauf. Hierbei bedeuten die Werte x1, x2 die Füllstände in zwei miteinander gekoppelten Gasspeichern (Hauptgasspeicher und Nebengasspeicher). Die Werte p1 und p2 bedeuten die Tragluftdruckwerte in den beiden Gasspeichern und der Wert "Gebläse" bedeutet die Gebläseleistung des Gebläses am zweiten Gasspeicher, den Nebengasspeicher, mit dem Füllstand x2 und dem Druck p2. Die Führungsgröße ist der Füllstand x1 im Hauptgasspeicher und die Regelgröße ist der Füllstand x2 im Nebengasspeicher. Die Regelabweichung ist somit der Füllstand x1 des Hauptgasspeichers minus dem Füllstand x2 des Nebengasspeichers.

Ist das Gebläse eingeschaltet (Leistung 100%) dann sinkt der Füllstand x2 des zweiten Gasspeichers relativ zum Füllstand x1 des ersten Gasspeichers, dem Hauptgasspeicher, d.h. dass Biogas aus dem zweiten Gasspeicher in den ersten Gasspeicher verdrängt wird. Dementsprechend kreuzen sich die Kurven zu den Füllständen x1, x2. Ist hingegen das Gebläse des zweiten Gasspeichers ausgeschaltet (Leistung 0%), dann nimmt der Tragluftdruck p2 des zweiten Gasspeichers binnen weniger Minuten ab, so dass Biogas aus dem ersten Gasspeicher in den zweiten Gasspeicher strömt und sich die Kurven der Füllstände x1, x2 wiederum in umgekehrter Richtung kreuzen. Durch diesen Gasausgleich zwischen den beiden Gasspeichern werden die Füllstände in beiden Gasspeichern einander angeglichen und auf etwa dem gleichen Niveau innerhalb der Regeltoleranz eingestellt. Hiermit lassen sich zwei Gasspeicher gleichzeitig auslasten.

Da der Hauptgasspeicher und der Nebengasspeicher über die Gasleitung 14 miteinander verbunden sind, folgt der Druck p1 des Hauptgasspeichers dem Druck p2 des Nebengasspeichers mit einem gewissen Versatz aufgrund des Strömungswiderstandes der Gasleitung 14 zwischen dem Hauptgasspeicher und dem Nebengasspeicher.

Bei dieser Regelung ist jedoch nachteilig, dass der Tragluftdruck kurzzeitig während das Gebläse ausgeschaltet ist auf sehr geringe Werte, von z.B. 1 mbar abfällt. So geringe Tragluftdrücke können bei starkem Wind dazu führen, dass die äußere Membran 8 verformt wird, wodurch der Betrieb beeinträchtigt werden kann.

Deshalb kann es vorteilhaft sein, ein Gebläse zu verwenden, das mit Teillast betrieben werden kann. Derartige Gebläse weisen üblicherweise einen Frequenz-Umformer auf und werden deshalb auch als FU-Gebläse bezeichnet. Figur 9 zeigt ein Diagramm des Betriebes zweier Gasspeicher, wobei der Gasspeicherfüllstand x2 auf den Gasspeicherfüllstand x1 durch Umschalten des Gebläses zwischen maximaler Leistung (100%) und reduzierter Leistung (80%) mit einem Totband von 3% geregelt wird.

Wie man diesem Diagramm entnehmen kann, schwankt der Tragluftdruck p2 um wenige mbar und ist immer deutlich größer als ein minimaler Tragluftdruck von 3 mbar. Beim Umschalten der Gebläseleistung auf reduzierte Leistung sinkt der Tragluftdruck p2 um etwa 1 mbar.

Mit einer solchen Regelung ist ein ausreichender Tragluftdruck sichergestellt, so dass auch bei starkem Wind die äußere Membran korrekt in ihrer Form gehalten wird.

Derartige FU-Gebläse unterliegen jedoch hohen Sicherheitsanforderungen und müssen mit einem speziellen Explosionsschutz versehen sein, so dass sie bei einer Undichtigkeit der inneren Membran keine Explosion am Gasspeicher auslösen.

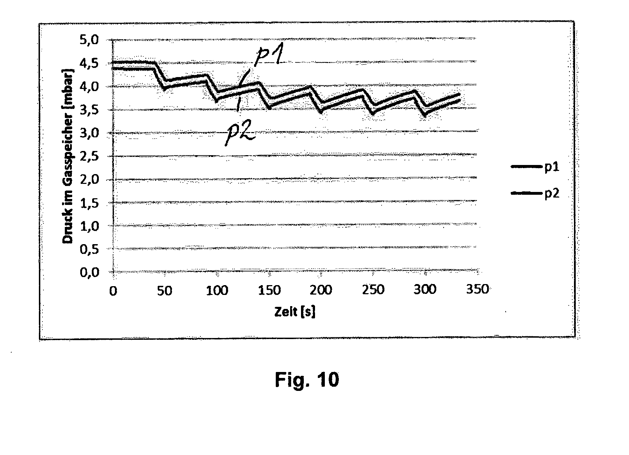

Da Gase kompressible Fluide sind, fällt beim Abschalten eines Gebläses der Druck nicht schlagartig sondern etwa exponentiell ab. Dies hat zur Folge, dass wenn man die Zeitdauer, während der das Gebläse abgeschaltet ist, kurz hält, der Tragluftdruck nur gering abfällt, so dass auch mit einem herkömmlichen Gebläse, das nur zwischen maximaler Leistung (100%) und keiner Leistung (0%) geschaltet werden kann, eine erfindungsgemäße Regelung einer Biogasanlage realisierbar ist, ohne dass der Tragluftdruck zu sehr abfällt. In Figur 10 ist der Verlauf der Tragluftdrücke p1 und p2 mit einer Ausschaltdauer von jeweils 10 s dargestellt. Die Einschaltdauer beträgt hierbei jeweils 40 s, so dass insgesamt eine Gebläseleistung von etwa 80% erzielt wird.

Bei dem in Figur 8 gezeigten Regelverfahren beträgt die Ausschaltzeit jeweils einige Minuten. Durch die Verkürzung der Ausschaltzeiten kann das Druckniveau des Tragluftdruckes auf einem ausreichenden Niveau gehalten werden. Es ist daher zweckmäßig, die Ausschaltzeit nicht länger als 30 s, insbesondere nicht länger als 20 s und vorzugsweise nicht länger als 15 s einzustellen. Mit einer solchen Regelung kann der Einsatz eines FU-Gebläses vermieden werden.

Im Rahmen der Erfindung ist es selbstverständlich auch möglich anstelle einer Zweipunktregelung eine andere Regelung, insbesondere einen PI-Regler zu verwenden. Dies macht vor allem Sinn in Verbindung mit einem FU-Gebläse, da hiermit die Füllstände und Drücke mit praktisch keinen Schwankungen geregelt werden können.

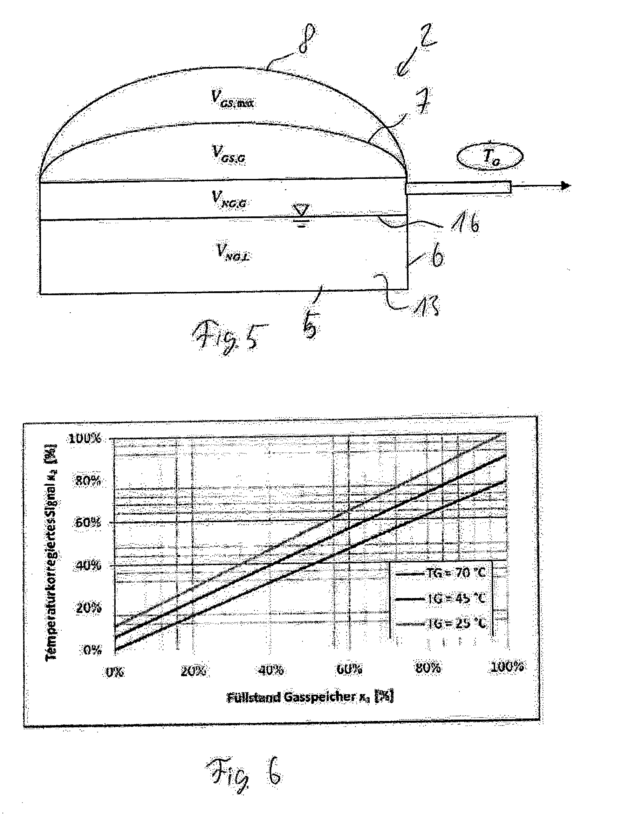

Nach einem zweiten Aspekt der vorliegenden Erfindung wird die Strömungsmenge des zum Verbraucher geführten Biogases in Abhängigkeit einer temperaturkorrigierten Füllmenge des Gasspeichers gesteuert. Figur 5 zeigt wiederum schematisch einen Doppelmembrangasspeicher 2 mit einer Bodenwandung 5, einer Mantelwandung 6, einer inneren Membran 7 und einer äußeren Membran 8. Der Gasspeicher ist mit einer Biomasse enthaltenen Suspension 13 gefüllt, die in ihrer Oberfläche eine Grenzfläche 16 zu dem darüber angeordneten Biogas bildet. Zwischen der inneren und äußeren Membran 7, 8 befindet sich Druckluft mit einem vorbestimmten von einem Gebläse einstellbaren Tragluftdruck.

Die Suspension hat ein Volumen von $V_{NG,L}$. Das Biogas umfasst einen unteren Abschnitt, der sich von der Grenzfläche 16 zwischen der Suspension 13 und dem Biogas bis zum oberen Rand der Mantelwandung 6 mit einem Volumen $V_{NG,G}$ erstreckt, und einen Abschnitt oberhalb des oberen Randes der Mantelwandung 6 und unterhalb der inneren Membran 7 mit dem Volumen $V_{GS,G}$. Bei maximalem Füllstand liegt die innere Membran 7 an der oberen Membran 8 an und der obere Volumenabschnitt des Biogases oberhalb des oberen Randes der Mantelwandung 6 erstreckt sich bis zur äußeren Membran 8 und hat das Volumen $V_{GS,max}$.

Ein Sensor, der die Höhe der inneren Membran 7 misst, erzeugt ein Signal, das proportional (Konstante: $x_1$) zum Volumen $V_{GS,G}$ ist:

$$V_{GS,G} = x_1 \cdot V_{GS,max}$$

Das Behältervolumen $V_{NG}$ zwischen der Bodenwandung 5 und dem oberen Rand der Mantelwandung 6 ist:

$$V_{NG} = V_{NG,L} + V_{NG,G}$$

Um den Verbraucher ansteuern zu können, wird eine Information benötigt, wieviel Gas tatsächlich auf der Anlage gespeichert ist. Diese Information wird auf einem Bereich von 0% bis 100% normiert.

Die maximale Gasmenge kann in der Biogasanlage gespeichert werden, wenn der Gasspeicher vollständig gefüllt ist und die Temperatur des Gases minimal ($T_{G,min}$) ist.

Die minimale Menge an Gas kann in der Biogasanlage gespeichert werden, wenn der Gasspeicher vollständig entleert und die Temperatur maximal ($T_{G,max}$) ist. Zwischen diesen beiden Extremen kann jede beliebige Gasmenge im System gespeichert werden. Die minimale Temperatur ist die kleinste Gastemperatur, die über einen Messzeitraum $t_m$ gemessen wurde. Die maximale Temperatur ($T_{G,max}$) ist die höchste Gastemperatur, die über den Messzeitraum $t_m$ gemessen wurde. Der Messzeitraum beträgt vorzugsweise zumindest einen oder mehrere Tage (z.B. eine Woche) und ist möglichst frei wählbar.

Die maximale Gasmenge $V_{G,max}$ berechnet sich aus:

$$V_{G,\max} = \frac{273K}{273K + T_{\min}}(V_{NG,G} + V_{GS,\max})$$

Die minimale Gasmenge $V_{G,min}$ berechnet sich aus:

$$V_{G,\min} = \frac{273K}{273K + T_{\max}} \cdot V_{NG,G}$$

Die aktuelle Gasmenge $V_G$ berechnet sich aus:

$$V_{G,\max} = \frac{273K}{273K + T_G}(V_{NG,G} + V_{GS,G})$$

Ein temperaturkorrigiertes Füllstandsignal $x_2$ ergibt sich aus einer linearen Interpolation:

$$x_2 = \frac{V_G - V_{G,\min}}{V_{G,\max} - V_{G,\min}}$$

Dieses temperaturkorrigierte Füllstandssignal kann zur Steuerung des Verbrauchers verwendet werden, ohne dass es zu temperaturbedingten Schwankungen kommt, da dieses Signal proportional zur Menge des Gases und nicht proportional zum tatsächlich vom Gas beanspruchten Volumen ist.

Im nachfolgenden Beispiel wird die Verwendung dieses temperaturkorrigierten Signals erläutert:

Ein Gasspeicher weist eine Wanne (Bodenwandung 5 und Mantelwandung 6) mit einem Volumen von 3570 m$^3$ auf. Diese Wanne kann jedoch nur bis zu einem Volumen von 3201 m$^3$ mit Suspension gefüllt werden. Der Gasspeicher ist zu 40% des maximal möglichen Füllvolumens mit Suspension gefüllt. Der Gasspeicher weist eine Doppelmembran mit einem Volumen $V_{GS,\max}$ = 2500 m$^3$ auf. Die Doppelmembran ist zu 70% mit Biogas gefüllt. Es wird angenommen, dass die Gasproduktion der Biogasanlage genau dem Gasverbrauch des Verbrauchers entspricht, so dass die Gasmenge im System konstant bleibt. Die Gastemperatur wird um 12.00 Uhr Mittags zu 65 °C gemessen. Die maximale Temperatur der letzten Woche betrug 70 °C. Die minimale Temperatur betrug 25 °C.

Es ergeben sich folgende Werte:

$$V_{GS,G} = 70\% \cdot 2500 \, m^3 = 1750 \, m^3 \qquad [1]$$

$$V_{NG,G} = \left(1 - 40\%\right) \cdot 3201 \, m^3 + \left(3570 \, m^3 - 3201 \, m^3\right) = 2289{,}6 \, m^3 \qquad [2]$$

Die minimalen, maximalen und tatsächlich gemessenen Gasmengen ergeben sich zu:

$$V_{G,\max} = \frac{273 \, K}{273 \, K + 25°C}\left(2289{,}6 \, m^3 + 2500 \, m^3\right) = 4388 \, Nm^3 \qquad [3]$$

$$V_{G,\min} = \frac{273 \, K}{273 \, K + 70°C} \cdot 2289{,}6 \, m^3 = 1822 \, Nm^3 \qquad [4]$$

$$V_G = \frac{273 \, K}{273 \, K + 65°C} \cdot \left(2289{,}6 \, m^3 + 1750 \, m^3\right) = 3276 \, Nm^3 \qquad [5]$$

Der temperaturkorrigierte Gasspeicherfüllstand ergibt sich zu

$$x_2 = \frac{3276\,Nm^3 - 1822\,Nm^3}{4388\,Nm^3 - 1822\,Nm^3} = 56{,}1\%$$

[6]

Fällt jetzt die Temperatur im Gasspeicher über Nacht auf $T_G$ = 30 °C, zieht sich die Gasphase zusammen. Das Normvolumen des Gases bleibt erhalten ($V_G$ = 3276 Nm³). Das Betriebsvolumen sinkt auf:

$$V_G = \frac{273\,K + 30°C}{273\,K} \cdot 3276\,Nm^3 = 3621\,m^3$$

Zieht man von diesem 3621 m³ das Volumen der Gasphase in der Wanne des Gasspeichers von 2289 m³ ab, ergibt sich ein Restvolumen von:

$$V_{G,GS} = 3621\,m^3 - 2289\,m^3 = 1332\,m^3$$

Der Füllstand in der Doppelmembran fällt somit auf:

$$x_1 = \frac{1332\,m^3}{2500\,m^3} = 53{,}3\%$$

Eine einfache Regelung, die nur den Füllstand der Doppelmembran zum Regeln nutzt, würde jetzt die Leistung des Gasverbrauchers reduzieren. Setzt man die neuen Bedingungen $x_1$ = 53,3% und $T_G$ = 30 °C in die Formeln [1] bis [6] ein, ergibt sich wieder der gleiche Füllstand der Doppelmembran von $x_2$ = 56,1%. Die Gastemperatur hat somit keinen Einfluss auf die Regelung des Gasverbrauchers.

Die Temperaturkorrektur hat jedoch Einfluss auf die Empfindlichkeit des Reglers. Der temperaturkorrigierte Füllstand kann nur noch 100% werden, wenn die Temperatur im Gasspeicher minimal wird. Umgekehrt kann der temperaturkorrigierte Füllstand nur noch auf 0% sinken, wenn die Gastemperatur maximal wird. Für eine Temperatur zwischen $T_{G,min}$ und $T_{G,max}$ kann das Signal nicht die Werte 0% und 100% erreichen (siehe Figur 6). Je geringer die Differenz zwischen $T_{G,min}$ und $T_{G,max}$ ist, desto sensitiver ist die Regelung des Gasverbrauchers. Aus diesem Grund darf der Meßzeitraum $t_m$ nicht zu groß gewählt, bzw. die Größen nicht von außen vorgegeben werden.

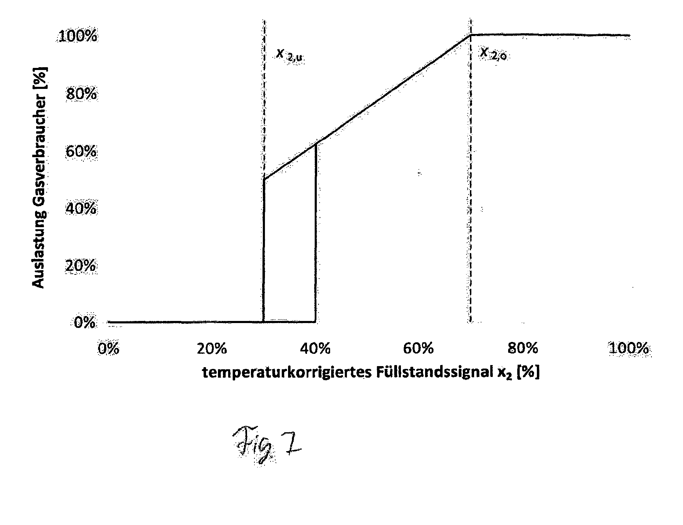

Bei der Einstellung einer Gasverbraucher-Kennlinie (Figur 7) müssen zwei Extremfälle berücksichtigt werden. Die Gasverbraucher-Kennlinie muss so gewählt werden, dass der temperaturkorrigierte Füllstand soweit steigen kann, dass der Gasverbraucher auf 100% Leistung laufen kann. Aus Figur 6 wird deutlich, dass bei Erreichen der maximalen Temperatur das temperaturkorrigierte Füllstandsignal maximal 76% anzeigen kann. Sollte die Gasverbraucherkennlinie so eingestellt sein, dass die maximale Leistung erst bei einem Füllstandsignal von 80% abgerufen wird, kann der Gasverbraucher nie mit voller Leistung betrieben werden, wenn die Temperatur nicht zu hoch ist (Figur 6).

Umgekehrt muss die Gasverbraucher-Kennline so eingestellt werden, dass bei Erreichen einer minimalen Gastemperatur das Füllstandsignal soweit absinkt, dass der Gasverbraucher noch abgeschaltet wird. Aus Figur 6 wird deutlich, dass bei Erreichen der minimalen Temperatur von 25 °C der temperaturkorrigierte Füllstand nur bis auf 10% absinken kann.

Die Regelung muss daher ständig überprüft werden, ob ein Zustand vorliegt, bei dem der Gasverbraucher nicht mehr auf Volllast oder vollständig abgeschaltet werden kann. Besonders bei niedrigen Flüssigkeits-Füllständen kann dies vorkommen, da der Atmungseffekt dann am größten ist.

Hierzu werden ein Einschaltfüllstand $x_{2,u}$ und ein Volllast-Füllstand $x_{2,o}$ definiert. Sie stellen das untere und obere Ende des linear ansteigenden Abschnitts in Figur 7 dar.

Die Formeln sind in Gleichung [7] und [8] widergegeben. Es ist auf die korrekte Verwendung von Normvolumen und Betriebsvolumen zu achten. $V_{NG,G}$ und $V_{GS,max}$ sind Betriebsvolumina [m³], Die restlichen Volumina in [7] und [8] sind Normvolumina [Nm³].

$$x_{2,u} \leq \frac{V_{NG,G} \cdot \dfrac{273\,K}{273\,K + T_G} - V_{G,\min}}{V_{G,\max} - V_{G,\min}} \qquad [7]$$

$$x_{2,o} \geq \frac{\left(V_{NG,G} + V_{GS,\max}\right) \cdot \dfrac{273K}{273K + T_G} - V_{G,\min}}{V_{G,\max} - V_{G,\min}} \qquad [8]$$

Sollte der Einschaltfüllstand $x_{2,u}$ nicht erreicht werden können (d.h. Gleichung [7] gilt nicht), so ist der Gasverbraucher abzuschalten. Sollte der Volllastfüllstand $x_{2,o}$ nicht erreicht werden können (d.h. Gleichung [8] gilt nicht), so muss der Gasverbraucher mit Volllast betrieben werden.

Für die Parametrierung der Gasverbraucherregelung können die Werte $x_{2,u}$ und $x_{2,o}$ so gewählt werden, dass ein Temperaturintervall von 20 bis 70°C keine Probleme in der Regelung bereitet. Der Werte werden beispielsweise bestimmt mit [9] und [10] zu:

$$x_{2,u} = \frac{\dfrac{V_{NG}}{273\,K + 20\,°C} - \dfrac{V_{NG}}{273\,K + 70\,°C}}{\dfrac{V_{NG} + V_{GS,\max}}{273\,K + 20\,°C} - \dfrac{V_{NG}}{273\,K + 70\,°C}} + 5\% \qquad [9]$$

$$x_{2,o} = \frac{\dfrac{V_{GS,\max}}{273\,K + 70\,°C}}{\dfrac{\left(V_{NG} + V_{GS,\max}\right)}{273\,K + 20\,°C} - \dfrac{V_{NG}}{273\,K + 70\,°C}} - 10\% \qquad [10]$$

Die Werte betragen im Beispiel $x_{2,u}$ =22,2% und $x_{2,o}$ = 60,7%. Im laufenden Betrieb können diese Werte an die Erfahrungswerte zur minimalen und maximalen Gastemperatur angepasst werden. Das Gas in als Endlager verwendeten Gasspeichern kann kälter werden, als das Gas in als Nachgär-Behältern verwendeten Gasspeichern.

Wenn die Biogasanlage so gefüttert wird, dass die Gasproduktion stets dem Gasverbrauch des Gasverbrauchers bei Volllast entspricht, wird sich im Gasspeicher ein Füllstand von etwas mehr als $x_{2,o}$ einstellen. Umgekehrt wird es mit der Temperaturkorrektur möglich sein, an der Änderung des Füllstands eine Über- oder Unterfütterung zu erkennen. Dies ist eine Voraussetzung für die Implementierung einer Fütterungsregelung.

Die hierbei verwendeten Parameter und Variablen sind:

$x_1$     [0..100%] Füllstand Gasspeicher

$T_G$     [°C] Gastemperatur

**berechnet**

$V_{NG,L}$ [m³]          Flüssigkeitsvolumen im Nachgärer

$V_{NG,G}$ [m³]    Gasvolumen im Nachgärer (zylindrischer Teil)
$V_G$ [Nm³]    Normvolumen gespeichertes Biogas
$V_{G,min}$ [Nm³]    minimales Normvolumen gespeichertes Biogas
$V_{G,max}$ [Nm³]    maximales Normvolumen gespeichertes Biogas
$T_{G,max}$ [°C]    maximale gemessene Gastemperatur im Meßzeitraum
$T_{G,min}$ [°C]    minimale gemessene Gastemperatur im Meßzeitraum
$x_2$ [0..100%]    Temperaturkorrigiertes Füllstandsignal
$x_{2,u}$ [%]    Unterer Einschaltwert Gasverbraucher
$x_{2,o}$ [%]    Oberer Schaltwert Volllast Gasverbraucher

**Konstanten** (vorgegeben)

$t_m$ [d]    Meßzeitraum, in dem minimale und maximale Temperatur bestimmt werden
$V_{NG}$ [m³]    Bruttovolumen des Nachgärers (zylindrischer Teil)
$V_{Gs,max}$ [m³]    maximales Volumen des Gasspeichers

Dieses temperaturkorrigierte Füllstandssignal kann auch in einer Biogasanlage mit mehreren Gasspeichern und der oben erläuterten Füllstandregelung mit einem Hauptgasspeicher und einem oder mehreren Nebengasspeichern verwendet werden.

Bei den oben erläuterten Ausführungsbeispielen sind alle Gasspeicher mit einer Doppelmembran ausgebildet. Im Rahmen der Erfindung ist es auch möglich, den Hauptgasspeicher anders auszubilden, beispielsweise mit einer EPDM-Membran oder einer starren Decke aus Beton, Stahl oder GFK oder einer beliebig anderen bekannten Bauweise für Gasspeicher.

**Bezugszeichenliste**

| | |
|---|---|
| 1 | Fermenter |
| 2 | Speicherbehälter/Gasspeicher |
| 3 | Rührwerk |
| 4 | Suspensionsrohrleitung |
| 5 | Bodenwandung |
| 6 | Mantelwandung |
| 7 | innere Membran |
| 8 | äußere Membran |
| 9 | Mittelsäule |
| 10 | Spanngurt |
| 11 | Messpunkt |
| 12 | Gewicht |
| 13 | Suspension |
| 14 | Gasleitung |
| 15 | Blockheizkraftwerk |
| 16 | Grenzfläche |
| 17 | Gasverbraucher-Kennlinie |

**Patentansprüche**

1. Biogasanlage mit
zumindest einem Fermenter (1) und zwei Speicherbehälter (2), wobei der Fermenter (1) und die beiden Speicherbehälter (2) kommunizierend miteinander verbunden sind, und die Speicherbehälter als Gasspeicher (2) ausgebildet sind und jeweils einen Füllstandssensor aufweisen, wobei einer der Gasspeicher (2) als Hauptgasspeicher definiert ist und der andere Gasspeicher (2) als Nebengasspeicher definiert ist, und zumindest der Nebengasspeicher als Doppelmembrangasspeicher ausgebildet ist, welcher eine innere Membran (7) und eine äußere Membran (8) aufweist, wobei ein Gebläse zum Einstellen eines Tragluftdruckes zwischen den beiden Membranen (7,8) vorgesehen ist,
einer Steuereinrichtung, die mit den Füllstandssensoren der Gasspeicher (2) und dem Gebläse des Doppelmembrangasspeichers verbunden und zur Regelung des Füllstandes im Nebengasspeicher ausgebildet ist, dass der Tragluftdruck derart eingestellt wird, dass der Füllstand im Nebengasspeicher dem Füllstand im Hauptgasspeicher entspricht, wobei der Füllstand im Nebengasspeicher die Regelgröße, der Füllstand im Hauptgasspeicher die Sollgröße und der Tragluftdruck die Stellgröße ist.

2. Biogasanlage nach Anspruch 1,
**dadurch gekennzeichnet,**
**dass** die Biogasanlage mehrere Nebengasspeicher (2) umfasst, wobei die Steuereinrichtung derart ausgebildet ist, dass der Tragluftdruck derart eingestellt wird, dass der Füllstand in allen Nebengasspeichern (2) dem Füllstand im Hauptgasspeicher (2) entspricht, wobei der Füllstand im jeweiligen Nebengasspeicher die Regelgröße, der Füllstand im Hauptgasspeicher die Sollgröße und der Tragluftdruck im jeweiligen Nebengasspeicher die Stellgröße ist.

3. Biogasanlage nach Anspruch 1 oder 2,
**dadurch gekennzeichnet,**
**dass** die Biogasanlage einen Verbraucher (15) aufweist, der mit zumindest einem der Gasspeicher (2) kommunizierend verbunden ist, damit dem Verbraucher (15) Biogas zugeführt werden kann, wobei der Druck im Hauptgasspeicher (2) derart einstellbar ist, dass das Biogas dem Verbraucher mit einem geringfügig größeren Druck als den Atmosphärendruck zugeführt, wobei der Druck am Verbraucher etwa 0,1 bis 4 mbar über dem Atmosphärendruck beträgt.

4. Biogasanlage nach einem der Ansprüche 1 bis 3,
**dadurch gekennzeichnet,**
**dass** die innere Membran des Doppelmembrangasspeichers mit einem Gewicht beaufschlagt ist.

5. Biogasanlage nach einem der Ansprüche 1 bis 4,
**dadurch gekennzeichnet,**
**dass** der Füllstandssensor zum Detektieren der Höhe eines bestimmten Punktes der inneren Membran (7) ausgebildet ist.

6. Biogasanlage nach einem der Ansprüche 1 bis 5, mit
zumindest einem Gasspeicher (2) und einem Verbraucher (15), der mit dem Gasspeicher (2) derart kommunizierend verbunden ist, dass dem Verbraucher (15) Biogas zugeführt werden kann, und eine Steuereinrichtung zum Steuern der Strömungsmenge des dem Verbraucher zuzuführenden Biogases derart ausgebildet ist, dass die Strömungsmenge in Abhängigkeit eines temperaturkorrigierten Füllstandes des Gasspeichers (2) gesteuert wird.

7. Biogasanlage nach Anspruch 6,
**dadurch gekennzeichnet,**
**dass** im Gasspeicher (2) oder in einer den Gasspeicher (2) mit dem Verbraucher (15) verbindenden Leitung ein Temperatursensor zum Messen der Gastemperatur angeordnet ist.

8. Biogasanlage nach einem der Ansprüche 3 bis 7;
**dadurch gekennzeichnet,**
**dass** der Verbraucher ein Blockheizkraftwerk (15), eine Bioerdgaseinspeisestation oder ein Gasbrenner ist.

9. Verfahren zum Betreiben einer Biogasanlage, die insbesondere nach einem der Ansprüche 1 bis 8 ausgebildet ist, wobei zumindest in einem Fermenter (1) Biogas erzeugt wird, das einem von zwei Gasspeichern (2) zugeführt wird, wobei die beiden Gasspeicher (2) kommunizierend miteinander verbunden sind, wobei einer der Gasspeicher (2) als Hauptgasspeicher definiert ist und der oder andere Gasspeicher als Nebengasspeicher definiert ist, und zumindest als Nebengasspeicher ein Doppelmembrangasspeicher verwendet wird, welcher eine innere Membran (7) und eine äußere Membran (8) aufweist, wobei ein Gebläse zum Einstellen eines Tragluftdruckes zwischen den beiden Membranen (7,8) vorgesehen ist,
und der Füllstand in beiden Gasspeichern (2) detektiert wird und der Tragluftdruck derart eingestellt wird, dass der Füllstand im Nebengasspeicher (2) dem Füllstand im Hauptgasspeicher (2) entspricht, wobei der Füllstand im Nebengasspeicher als Regelgröße, der Füllstand im Hauptgasspeicher als Sollgröße und der Tragluftdruck als Stellgröße verwendet wird.

**10.** Verfahren nach Anspruch 9,
**dadurch gekennzeichnet,**
**dass** als Hauptgasspeicher (2) auch ein Doppelmembrangasspeicher mit einer inneren und einer äußeren Membran (7,8) verwendet wird, wobei vorzugsweise ein vorbestimmter Tragluftdruck derart eingestellt wird, dass an einem an die Biogasanlage angeschlossenen Verbraucher (15) das Biogas mit einem Druck geringfügig über Atmosphärendruck anliegt.

**11.** Verfahren zum Betreiben einer Biogasanlage nach einem der Ansprüche 9 oder 10, und insbesondere zum Betreiben einer Biogasanlage nach einem der Ansprüche 1 bis 7,
wobei zumindest in einem Gasspeicher (2) Biogas gespeichert wird und aus dem Gasspeicher (2) einem Verbraucher (15) zugeführt wird, und die Strömungsmenge des dem Verbraucher (15) zugeführten Biogases in Abhängigkeit eines temperaturkorrigierten Füllstandes des Gasspeichers gesteuert wird.

**12.** Verfahren nach Anspruch 11,
**dadurch gekennzeichnet,**
**dass** bei Unterschreiten einer vorbestimmten temperaturkorrigierte Füllmenge des Gasspeichers die Strömungsmenge reduziert wird.

**13.** Verfahren nach Anspruch 11 oder 12,
**dadurch gekennzeichnet,**
**dass** der temperaturkorrigierte Füllstand ($x_2$) mit einer lineare Interpolation aus der maximalen Gasmenge ($V_{G,max}$) und der minimalen Gasmenge ($V_{G,min}$), die in der Biogaslanlage gespeichert werden können, berechnet wird.

**14.** Verfahren nach Anspruch 13,
**dadurch gekennzeichnet,**
**dass** die maximalen Gasmenge ($V_{G,max}$) bei einer minimalen Temperatur ($T_{G,min}$) des Gases innerhalb eines bestimmten Messzeitraumes und die minimalen Gasmenge ($V_{G,min}$) bei einer maximalen Temperatur ($T_{G,max}$) des Gases innerhalb eines bestimmten Messzeitraumes, berechnet wird.

**15.** Verfahren nach einem der Ansprüche 9 bis 14,
**dadurch gekennzeichnet,**
**dass** der Tragluftdruck im Nebengasspeicher mit einem Gebläse eingestellt wird, das nur zwischen einer maximalen Gebläseleistung und keiner Gebläseleistung (= ausgeschaltet) geschaltet werden kann, wobei die Zeitdauer während der das Gebläse ausgeschaltet ist (keine Gebläseleistung) maximal 30 s, vorzugsweise maximal 15 s, beträgt.

**Claims**

**1.** Biogas plant with
at least one fermenter (1) and two storage vessels (2), wherein the fermenter (1) and the two storage vessels (2) are connected so as to communicate with one another, and the storage vessels are in the form of gas holders (2) with each having a fill level sensor, wherein one of the gas holders (2) is defined as the main gas holder and the other gas holder (2) is defined as secondary gas holder, and at least the secondary gas holder is in the form of a double membrane gas holder which has an inner membrane (7) and an outer membrane (8), wherein a fan is provided to set a supporting air pressure between the two membranes (7, 8),
a control device which is connected to the fill level sensors of the gas holders (2) and the fan of the double membrane gas holder and is designed to regulate the fill level in the secondary gas holder so that the supporting air pressure is set so that the fill level in the secondary gas holder corresponds to the fill level in the main gas holder, wherein the fill level in the secondary gas holder is the control variable, the fill level in the main gas holder is the specified variable, and the supporting air pressure is the correcting variable.

**2.** Biogas plant according to claim 1,
**characterised in that**
the biogas plant comprises several secondary gas holders (2), wherein the control device is so designed that the supporting air pressure is set in such a way that the fill level in all secondary gas holders (2) corresponds to the fill level in the main gas holder (2), wherein the fill level in the secondary gas holder is the control variable, the fill level in the main gas holder is the specified variable, and the supporting air pressure in the respective secondary gas holder is the correcting variable.

**3.** Biogas plant according to claim 1 or 2,
**characterised in that**
the biogas plant has a load (15) which is connected so as to communicate with at least one of the gas holders (2) so that biogas may be fed to the load (15), wherein the pressure in the main gas holder may be set so that the biogas is fed to the load with a slightly greater pressure than the atmospheric pressure, wherein the pressure at the load is around 0.1 to 4 mbar above atmospheric pressure.

**4.** Biogas plant according to any of claims 1 to 3,
**characterised in that**
the inner membrane of the double membrane gas holder is loaded by a weight.

**5.** Biogas plant according to any of claims 1 to 4,
**characterised in that**
the fill level sensor is designed to detect the height of a specific point of the inner membrane (7).

**6.** Biogas plant, according to any of claims 1 to 5, with
at least one gas holder (2) and one load (15) which is connected so as to communicate with the gas holder (2) so that biogas may be fed to the load (15), and a control device to control the flow volume of the biogas to be fed to the load is so designed that the flow volume is controlled depending on a temperature-corrected fill level of the gas holder (2).

**7.** Biogas plant according to claim 6,
**characterised in that**
a temperature sensor for measuring the gas temperature is provided in the gas holder (2) or in a line connecting the gas holder (2) to the load (15).

**8.** Biogas plant according to any of claims 3 to 7,
**characterised in that**
the load is a combined heat and power unit (15), a bio natural gas feed station or a gas burner.

**9.** Method of operating a biogas plant, designed in particular according to any of claims 1 to 8,
wherein biogas is produced in at least one fermenter (1) and fed to one of two gas holders (2), wherein the two gas holders (2) are connected so as to communicate with one another, wherein one of the gas holders (2) is defined as the main gas holder and the other gas holder is defined as secondary gas holder, and at least the secondary gas holder is in the form of a double membrane gas holder which has an inner membrane (7) and an outer membrane (8), wherein a fan is provided to set a supporting air pressure between the two membranes (7, 8), and the fill level in both gas holders (2) is detected and the supporting air pressure is set in such a way that the fill level in the secondary gas holder (2) corresponds to the fill level in the main gas holder (2), wherein the fill level in the secondary gas holder is the control variable, the fill level in the main gas holder is the specified variable, and the supporting air pressure is the correcting variable.

**10.** Method according to claim 9,
**characterised in that**
a double membrane gas holder with an inner and an outer membrane (7, 8) is also used as main gas holder (2), wherein preferably a predetermined supporting air pressure is set in such a way that the biogas is provided to the load (15) connected to the biogas plant at a pressure slightly above atmospheric pressure.

**11.** Method of operating a biogas plant, according to one of claims 9 or 10, and in particular for operating a biogas plant according to any of claims 1 to 7, wherein at least in one gas holder (2) biogas is stored and is fed from the gas holder (2) to a load (15), and the flow volume of the biogas fed to the load (15) is controlled depending on a temperature-corrected fill level of the gas holder.

**12.** Method according to claim 11,
**characterised in that**
the flow volume is reduced if the gas holder fill level falls below a predetermined temperature-corrected value.

**13.** Method according to claim 11 or 12,
**characterised in that**

the temperature-corrected fill level ($X_2$) is calculated by a linear interpolation from the maximum gas quantity ($V_{G,max}$) and the minimum gas quantity ($V_{G,min}$) which may be stored in the biogas plant.

14. Method according to claim 13,
   **characterised in that**
   the maximum gas quantity ($V_{G,max}$) is calculated at a minimum temperature ($T_{G,min}$) of the gas within a defined period of measurement, and the minimum gas quantity ($V_{G,min}$) at a maximum temperature ($T_{G,max}$) of the gas within a defined period of measurement.

15. Method according to any of claims 9 to 14,
   **characterised in that**
   the supporting air pressure in the secondary gas holder is adjusted by a fan which may be switched only between a maximum fan output and no fan output (= switched off), wherein the length of time during which the fan is switched off (no fan output) is a maximum 30 secs., preferably a maximum 15 secs.

## Revendications

1. Installation de biogaz, comprenant
   au moins un fermenteur (1) et deux récipients de stockage (2), dans laquelle le fermenteur (1) et les deux récipients de stockage (2) sont reliés en communication les uns avec les autres, et les récipients de stockage sont réalisés sous forme d'accumulateurs de gaz (2) et comprennent chacun un capteur de niveau de remplissage, dans laquelle l'un des accumulateurs de gaz (2) est défini comme accumulateur de gaz principal et l'autre accumulateur de gaz (2) est défini comme accumulateur de gaz annexe, et au moins l'accumulateur de gaz annexe est réalisé sous forme d'accumulateur de gaz à double membrane, qui comprend une membrane intérieure (7) et une membrane extérieure (8), et il est prévu un ventilateur pour établir une pression d'air portante entre les deux membranes (7, 8),
   un dispositif de commande, qui est connecté aux capteurs de niveau de remplissage des accumulateurs de gaz (2) et au ventilateur de l'accumulateur de gaz à double membrane, et qui est réalisé pour réguler le niveau de remplissage dans l'accumulateur de gaz auxiliaire, dans laquelle la pression d'air portante est réglée de telle façon que le niveau de remplissage de l'accumulateur de gaz auxiliaire correspond au niveau de remplissage dans l'accumulateur de gaz principal, et le niveau de remplissage dans l'accumulateur de gaz auxiliaire est la grandeur à réguler, le niveau de remplissage dans l'accumulateur de cas principal est la grandeur de consigne, et la pression d'air portante est la grandeur de réglage.

2. Installation de biogaz selon la revendication 1,
   **caractérisée en ce que** l'installation de biogaz comprend plusieurs accumulateurs de gaz auxiliaires (2), et le dispositif de commande est réalisé de telle façon que la pression d'air portante est réglée de manière à ce que le niveau de remplissage dans tous les accumulateurs de gaz auxiliaires (2) corresponde au niveau de remplissage dans l'accumulateur de gaz principal (2), et dans laquelle le niveau de remplissage dans l'accumulateur de gaz auxiliaire respectif est la grandeur à réguler, le niveau de remplissage dans l'accumulateur de gaz principal est la grandeur de consigne, et la pression d'air portante dans l'accumulateur de gaz auxiliaire respectif est la grandeur de réglage.

3. Installation de biogaz selon la revendication 1 ou 2,
   **caractérisée en ce que** l'installation de biogaz comprend un appareil consommateur (15) qui est relié en communication avec l'un au moins des accumulateurs de gaz (2) afin de pouvoir amener du biogaz à l'appareil consommateur (15), et la pression dans l'accumulateur de gaz principal (2) est réglable de telle façon que le biogaz est amené à l'appareil consommateur avec une pression légèrement plus élevée que la pression atmosphérique, de sorte que la pression au niveau de l'appareil consommateur s'élève environ à 0,1 à 4 mbar au-dessus de la pression atmosphérique.

4. Installation de biogaz selon l'une des revendications 1 à 3,
   **caractérisée en ce que** la membrane intérieure de l'accumulateur de gaz à double membrane est sollicitée avec un poids.

5. Installation de biogaz selon l'une des revendications 1 à 4,
   **caractérisée en ce que** le capteur de niveau de remplissage est réalisé pour détecter la hauteur d'un point déterminé de la membrane intérieure (7).

**6.** Installation de biogaz selon l'une des revendications 1 à 5, comprenant au moins un accumulateur de gaz (2) et un appareil consommateur (15), qui est relié en communication avec l'accumulateur de gaz (2) afin de pouvoir amener du biogaz à l'appareil consommateur (15), et un dispositif de commande pour commander la quantité du biogaz à amener à l'appareil consommateur est réalisé de telle manière que la quantité est commandée en fonction d'un niveau de remplissage de l'accumulateur de gaz (2).

**7.** Installation de biogaz selon la revendication 6, **caractérisée en ce qu'**un capteur de température destiné à mesurer la température du gaz est agencé dans l'accumulateur de gaz (2) ou dans un conduit qui relie l'accumulateur de gaz (2) à l'appareil consommateur (15).

**8.** Installation de biogaz selon l'une des revendications 3 à 7, **caractérisée en ce que** l'appareil consommateur est une centrale de chauffage combinée (15), une station d'injection de gaz naturel biologique ou un brûleur à gaz.

**9.** Procédé pour le fonctionnement d'une installation de biogaz, qui est réalisée en particulier selon l'une des revendications 1 à 8, dans lequel on engendre du biogaz dans au moins un fermenteur (1), le biogaz est amené à un accumulateur de gaz (2) parmi deux accumulateurs de gaz, lesdits deux accumulateurs de gaz (2) sont reliés en communication l'un avec l'autre, tels que l'un des accumulateurs de gaz (2) est défini comme accumulateur de gaz principal et l'autre accumulateur de gaz est défini comme accumulateur de gaz auxiliaire, et on utilise au moins à titre d'accumulateur de gaz auxiliaire un accumulateur de gaz à double membrane qui comprend une membrane intérieure (7) et une membrane extérieure (8), dans lequel il est prévu un ventilateur pour régler une pression d'air portant entre les deux membranes (7, 8),
et le niveau de remplissage dans les deux accumulateurs de gaz (2) est détecté et la pression de gaz portante est réglée de telle manière que le niveau de remplissage dans l'accumulateur de gaz annexe (2) correspond au niveau de remplissage dans l'accumulateur de gaz principal (2), et le niveau de remplissage dans l'accumulateur de gaz annexe est utilisé comme grandeur à réguler, le niveau de remplissage dans l'accumulateur de gaz principal est utilisé comme grandeur de consigne, et la pression d'air portante est utilisée comme grandeur de réglage.

**10.** Procédé selon la revendication 9, **caractérisée en ce que** l'on utilise comme accumulateur de gaz principal (2) également un accumulateur de gaz à double membrane avec une membrane intérieure et une membrane extérieure (7, 8), et on règle de préférence une pression d'air portante prédéterminée de telle manière que le biogaz est alimenté avec une pression légèrement au-dessus de la pression atmosphérique dans un appareil consommateur (15) branché à l'installation de biogaz.

**11.** Procédé pour le fonctionnement d'une installation de biogaz selon l'une des revendications 9 ou 10, et en particulier pour le fonctionnement d'une installation de biogaz selon l'une des revendications 1 à 7, dans lequel on stocke du biogaz dans au moins un accumulateur de gaz (2) et on l'amène depuis l'accumulateur de gaz (2) à un appareil consommateur (15), et la quantité du biogaz amené à l'appareil consommateur (15) est commandée en fonction d'un niveau de remplissage de l'accumulateur de gaz.

**12.** Procédé selon la revendication 11, **caractérisé en ce que** la quantité est réduite lorsqu'on passe au-dessous d'une quantité de remplissage prédéterminée corrigée en température de l'accumulateur de gaz.

**13.** Procédé selon la revendication 11 ou 12, **caractérisé en ce que** le niveau de remplissage corrigé en température ($X_2$) est calculé avec une interpolation linéaire à partir de la quantité de gaz maximale ($V_{G,max}$) et de la quantité de gaz minimale ($V_{G,min}$), qui peuvent être stockées dans l'installation de biogaz.

**14.** Procédé selon la revendication 13, **caractérisé en ce que** la quantité de gaz maximale ($V_{G,max}$) est calculée pour une température minimale ($T_{G,min}$) du gaz à l'intérieur d'un intervalle temporel de mesure déterminé, et la quantité de gaz minimale ($V_{G,min}$) pour une température maximale ($T_{G,max}$) du gaz à l'intérieur d'un intervalle temporel de mesure déterminé.

**15.** Procédé selon l'une des revendications 9 à 14, **caractérisé en ce que** la pression d'air portante dans l'accumulateur de gaz auxiliaire est réglée avec un ventilateur, qui est susceptible d'être commuté entre une puissance de ventilation maximale et aucune puissance de ventilation (= à l'arrêt), et la durée temporelle pendant laquelle le ventilateur est à l'arrêt (aucune puissance de ventilation) est au maximum 30 secondes, de préférence au maximum 15 secondes.

Fig. 1

1/1

1/2

2/2

2/3

2/1

P(1) = 0,669 mbar

P(2) = 0,659 mbar

P(3) = 0,382 mbar

P(4) = 0,388 mbar

P(5) = 0,367 mbar

P(6) = 0,339 mbar

P(7) = 0,237 mbar

P(8) = 0,000 mbar

V(1-2) = 22,8 Nm³/h

V(1-3) = 652,2 Nm³/h

V(2-4) = 697,8 Nm³/h

V(3-4) = -102,8 Nm³/h

V(4-5) = 203,6 Nm³/h

V(4-6) = 459, Nm³/h

V(5-6) = 218,6 Nm³/h

V(3-7) = 822,5 Nm³/h

V(6-7) = 677,5 Nm³/h

V(7-8) = 1500, Nm³/h

Fig. 2

22

Fig. 3

Fig. 4c
Fig. 4a
Fig. 4d
Fig. 4b
Fig. 4e
Fig. 4f

$V_{GS,max}$

$V_{GS,G}$

$V_{NG,G}$

$V_{NG,1}$

$T_G$

Fig. 5

Fig. 6

*Fig 7*

**Fig. 8**

**Fig. 9**

Fig. 10

**EP 2 535 402 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9958458 A
- DE 20022768 U1
- DE 102008031882 A1
- WO 2010054827 A1